# EUROPEAN PATENT SPECIFICATION

(11) **EP 0 674 635 B1**
(45) Date of publication and mention of the grant of the patent: **28.03.2001**
(21) Application number: 94902924.3
(22) Date of filing: 21.12.1993
(51) Int. Cl.: C07D 453/02, A61K 31/435

(54) **QUINUCLIDINE DERIVATIVES AS SQUALENE SYNTHASE INHIBITORS**
CHINUCLIDIN DERIVATE ALS SQUALEN SYNTHASE INHIBITOREN
DERIVES DE QUINUCLIDINE COMME INHIBITEURS DE LA SQUALENE SYNTHETASE

(30) Priority: 21.12.1992 GB 9226574; 21.12.1992 GB 9226576
(43) Date of publication of application: 04.10.1995
(73) Proprietor: Syngenta Limited, Haselmere, Surrey GU27 3JE (GB)
(72) Inventor: BROWN, George Robert, Wilmslow, Cheshire SK9 6HH (GB); MALLION, Keith Blakeney, Knutsford, Cheshire WA16 8AE (GB)
(74) Representative: Bill, Kevin
(86) International application number: GB9302614
(87) International publication number: WO9414803

(56) References cited:
- EP-A- 0 307 142
- WO-A-92/15579
- WO-A-93/09115
- WO-A-93/24486
- US-A- 5 135 935

## Description

This invention concerns heterocyclic derivatives which are useful in inhibiting squalene synthase, processes for their preparation and pharmaceutical compositions containing them. The present invention is also concerned with methods of using such heterocyclic derivatives in treating diseases and medical conditions where inhibition of squalene synthase is desirable, for example in treating diseases or medical conditions such as hypercholesterolemia and atherosclerosis.

Several different classes of compounds have been reported to possess the capability of being able to lower cholesterol levels in blood plasma. For example agents which inhibit the enzyme HMG CoA reductase, which is essential for the production of cholesterol, have been reported to reduce levels of serum cholesterol. Illustrative of this class of compounds is the HMG CoA reductase inhibitor known as lovastatin which is disclosed in US Patent No 4,231,938. Other agents which are reported to lower serum cholesterol include those which act by complexing with bile acids in the intestinal system and which are hence termed "bile acid sequestrants". It is believed that many of such agents act by sequestering bile acids within the intestinal tract. This results in a lowering of the levels of bile acid circulating in the enteroheptatic system and promoting replacement of bile acids by synthesis in the liver from cholesterol, which results in an upregulation of the hepatic LDL receptor and hence in a lowering of circulating blood cholesterol levels. Squalene synthase (also referred to in the art as squalene synthetase) is a microsomal enzyme which catalyses the first committed step of cholesterol biosynthesis. Two molecules of farnesyl pyrophosphate (FPP) are condensed in the presence of the reduced form of nicotinamide adenine dinucleotide phosphate (NADPH) to form squalene. The inhibition of this committed step to cholesterol should leave unhindered biosynthetic pathways to ubiquinone, dolichol and isopentenyl t-RNA. Elevated cholesterol levels are known to be one of the main risk factors for ischaemic cardiovacsular disease. Thus, an agent which inhibits squalene synthase should be useful in treating diseases and medical conditions in which a reduction in the levels of cholesterol is desirable, for example hypercholesterolemia and atherosclerosis.

Thus far, the design of squalene synthase inhibitors has concentrated on the preparation of analogues of the substrate farnesyl pyrophosphate (FPP), and hence on compounds which contain phosphorus groups. For example, the preparation of phosphorous-containing squalene synthase inhibitors is reported in published European Patent Application No. 409,181; and the preparation of isoprenoid (phosphinylmethyl)phosphonates as inhibitors of squalene synthase is reported by Biller et al, J. Med. Chem., 1988, 31, 1869. Various quinuclidine derivatives have been reported in, for example, EP 307,142 and EP 316,718 to be muscarinic agonists. Recently certain quinuclidine derivatives have been reported (WO 92/15579 and US 5,135,935) to inhibit squalene synthase.

The present invention is based on the discovery that certain heterocyclic derivatives are inhibitors of squalene synthase, and are hence useful in treating diseases and medical conditions in which inhibition of squalene synthase is desirable.

According to the present invention there is provided a compound of formula I (formula set out hereinafter together with the other chemical formulae referred to herein), or a pharmaceutically acceptable salt thereof, wherein:
R¹ is hydrogen or hydroxy;
R² is hydrogen; or
R¹ and R² are joined together so that CR¹-CR² is a double bond;
Ar¹ is a phenylene moiety and Ar² is a heterocyclic moiety selected from an aromatic 5-membered or 6-membered heterocyclic ring containing one, two or three heteroatoms selected from nitrogen, oxygen and sulphur; an aromatic 5-membered or 6-membered heterocyclic ring containing one, two or three heteroatoms selected from nitrogen, oxygen and sulphur which is fused to a benzene ring; and a bicyclic heterocycle which consists of a non-aromatic 5-membered or 6-membered heterocyclic ring containing (in addition to carbon atoms) one, two or three heteroatoms selected from nitrogen, oxygen and sulphur fused to a benzene ring; and wherein one or both of Ar¹ and Ar² may optionally bear one or more substituents independently selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino N-(1-6C)alkylcarbamoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno (1-6C)alkyl, (1-6C)alkanoylamino, ureido, N'-(1-6C)alkylureido, (1-6C)alkanoyl and oxime derivatives thereof and O-(1-6C)alkyl ethers of said oxime derivatives; provided that Ar² is not a 6-membered heteroaryl moiety containing one or two nitrogen atoms.

It will be understood that when formula I compounds contain a chiral centre, the compounds of the invention may exist in, and be isolated in, optically active or racemic form. The invention includes any optically active or racemic form of a compound of formula I which possesses the beneficial pharmacological effect of inhibiting squalene synthase. The synthesis of optically active forms may be carried out by standard techniques of organic chemistry well known in the art, for example by, resolution of a racemic form, by synthesis from optically active starting materials or by asymmetric synthesis.

It will also be understood that, insofar as certain of the compounds of the formula I may exhibit the phenomenon of tautomerism, for example a compound of formula I in which Ar² bears a hydroxy substituent, the present invention includes any tautomeric form of a compound of formula I which possesses the beneficial pharmacological effect of inhibiting squalene synthase.

It is also to be understood that generic terms such as "alkyl" include both the straight chain and branched chain groups such as butyl and tert-butyl. However, when a specific term such as "butyl" is used, it is specific for the straight chain or "normal" butyl group, branched chain isomers such as "t-butyl" being referred to specifically when intended.

It will also be appreciated that oxime derivatives of the (1-6C)alkanoyl group will comprise aldoximes and ketoximes of formula -C(Ra)=NOH (Ra is H or alkyl), and the O-alkyl ethers of these oximes will have the formula -C(Ra)=NORb (Ra is H or alkyl, and Rb is alkyl).

It will be appreciated that when R¹ and R² are joined so that CR¹⁻CR² is a double bond, the quinuclidine ring in formula I will comprise the 2,3-dehydroquinuclidine moiety shown in formula Ia.

It will be appreciated that Ar² may be attached to Ar¹ through any available ring atom.

Suitable values for Ar¹, when Ar¹ is a phenylene moiety, include 1,2-phenylene, 1,3-phenylene and 1,4-phenylene.

A particular value for an optional substituent which may be present on Ar¹ or Ar² is, for example,
- for alkyl;: (1-4C)alkyl, such as methyl, ethyl, propyl, isopropyl, butyl, isobutyl or sec-butyl;
- for alkenyl;: (2-4C)alkenyl, such as allyl, prop-2-enyl, but-2-enyl or 2-methyl-2-propenyl;
- for alkynyl;: (2-4C)alkynyl, such as prop-2-ynyl or but-2-ynyl;
- for alkoxy;: (1-4C)alkoxy, such as methoxy, ethoxy, propoxy, isopropoxy or butoxy;
- for alkylamino;: (1-4C)alkylamino, such as methylamino, ethylamino, propylamino or butylamino;
- for di-alkylamino;: di-[(1-4C)alkylamino, such as dimethylamino, diethylamino, methylpropylamino or dipropylamino;
- for alkylcarbamoyl;: N-methylcarbamoyl, N-ethylcarbamoyl or N-propylcarbamoyl;
- for di-alkylcarbamoyl;: N,N-dimetllylcarbmnoyl or N,N-diethylcarbamoyl;
- for alkoxycarbonyl;: methoxycarbonyl, ethoxycarbonyl or propoxycarbonyl;
- for alkylthio;: methylthio, ethylthio, propylthio, isopropylthio or butylthio;
- for alkylsulphinyl;: methylsulphinyl, ethylsulphinyl, propylsulphinyl, isopropylsulphinyl or butylsulphinyl;
- for alkylsulphonyl;: methylsulphonyl, ethylsulphonyl, propylsulphonyl, isoproylsulphonyl or butylsulphonyl;
- for halogeno;: fluoro, chloro, bromo or iodo;
- for halogenoalkyl;: halogenoalkyl containing one, two or three halo groups selected from fluoro, chloro, bromo and iodo and an alkyl group selected from methyl, ethyl, propyl, iso-propyl, butyl, iso-butyl and sec-butyl, (in particular fluoromethyl, difluoromethyl or trifluoromethyl);
- for alkylureido;: N'-methylureido, N'-ethylureido, N'-prpoylureido, N'-isopropylureido or N'-butylureido;
- for alkanoyl;: formyl, acetyl, propionyl and butyryl;
- for O-(1-6C)alkyl: methyl, ethyl, propyl, isopropyl and butyl esters ethers of alkanoyl of said oximes; and oximes;
- for alkanoylamino;: formamido, acetamido, propionamido, iso-propionamido, butyramido or iso-butyramido.

A particular value for Ar¹ when Ar¹ is a phenylene moiety is, for example, 1,3-phenylene or 1,4-phenylene.

Particular values for Ar² when Ar² is a heterocyclic moiety include, for example, furyl, pyrrolyl, thienyl, imidazolyl, oxazolyl, isooxazolyl, thiazolyl, isothiazolyl, oxadiazolyl, 1,2,3-triazolyl, 1,2,4-triazolyl, benzfuranyl, quinolyl, isoquinolyl, benzimidazolyl, indolyl, benzthiazolyl, benzdioxanyl (such as 1,4-benzdioxanyl), benzdioxolyl (such as 1,3-benzdioxolyl) and 2,3-dihydrobenzfuranyl.

More particular values for Ar² when it represents a heterocyclic moiety include, for example, pyrrolyl, quinolyl, oxadiazolyl, oxazolyl, thienyl, thiazolyl, furyl, pyridyl, benzthiazolyl, benzoxazolyl, benzimidazolyl and benzdioxolyl.

Values of particular interest for Ar² when Ar² is a heterocyclic moiety include, for example, pyrrolyl, quinolyl, oxadiazolyl, oxazolyl, thienyl, thiazolyl, furyl, pyridyl, benzthiazolyl, benzoxazolyl, benzimidazolyl and benzdioxolyl, especially pyrrolyl, quinolyl, oxadiazolyl, oxazolyl, thiazolyl, benzthiazolyl and benzdioxolyl (more especialy oxadiazolyl or quinoyl).

In general, it is preferred that Ar¹ is optionally unsubstituted or substituted by one, two or three substituents independently selected from those mentioned above; and that Ar² is optionally unsubstituted or substituted by one, two or three substituents from those mentioned above.

In a particular embodiment, one or both of Ar¹ and Ar² may optionally bear one or more substituents selected from halogeno, hydroxy, nitro, (1-6C)alkyl, (2-6C)alkenyl, (1-6C)alkoxy, (1-6C)alkanoylamino, halogeno-(1-6C)alkyl, (1-6C)alkanoyl and oxime derivatives thereof and O-(1-6C)alkyl ethers of said oximes.

In general it is preferred, for example, that R¹ is hydroxy and R² is hydrogen.

In general it is preferred, for example, that when Ar¹ is phenylene , Ar¹ is 1,4-phenylene.

It is generally preferred, for example, that Ar2 is a heterocyclic moiety selected from an aromatic 5-membered or 6-membered heterocyclic ring containing (in addition to carbon atoms) one, two or three heteroatoms selected from nitrogen, oxygen and sulphur which is optionally fused to a benzene ring.

It is generally preferred, for example, that when Ar² is a heterocyclic moiety it is an oxadiazole or quinolyl moiety.

Specific values for R¹/R² include when R¹ is hydroxy and R² is hydrogen.

Specific values for -Ar¹-Ar² include, for example, a 4-(2,5-dimethylpyrrol-1-yl)phenyl, 4-(1-pyrrolyl)phenyl, 4-(2-quinolyl)phenyl, 4-(2-benzthiazolyl)phenyl, 4-(3-methyl-1,2,4-oxadiazol-5-yl)phenyl, 4-(2-benzoxazolyl)phenyl, 4-(2-methyloxazol-5-yl)phenyl, 4-(1-meythl-benzimidazol-2-yl)phenyl, 4-(2-thienyl)phenyl, 4-(2-thiazolyl)phenyl, 4-(3,4-methylenedioxyphenyl)phenyl, 4-(5-methyl-1 ,2,4-oxadiazol-3-yl)phenyl, 4-(3-quinolyl)phenyl, 4-(3-thienyl)phenyl or a 4-(3-furyl)phenyl moiety.

Further values of interest for Ar² include, for example, 2-thienyl, 1-imidazolyl, 1-pyrrolyl, 2-imidazolyl, 1,2,3-triazol-1-yl, 1,2,3-triazol-3-yl, 3-quinolyl, 2-quinolyl, 1-isoquinolyl, 2-thiazolyl, 3-isoquinolyl, 1-benzimidazolyl, 2-benzthiazolyl, 5-oxadiazoyl, 2-indolyl and 3-indolyl, which may optionally bear one or two substituents independently selected from fluoro, chloro, bromo, iodo, hydroxy, nitro, methyl, ethyl, propyl, isopropyl, butyl, isobutyl, sec-butyl, allyl, methoxy, ethoxy, propoxy, isopropoxy, butoxy, acetamido, propionamido, iso-propionamido, fluoromethyl, difluoromethyl, trifluoromethyl, formyl, acetyl, propionyl, butyryl and oxime derivatives of the last four groups and O-methyl, ethyl, propyl, isopropyl and butyl ethers of said oximes.

Further values of interest for Ar¹ include, for example, an unsubstituted 1,4-phenylene moiety and a 1,4-phenylene moiety having one or two substituents independently selected from fluoro, chloro, bromo, iodo, hydroxy, nitro, methyl, ethyl, propyl, isopropyl, butyl, isobutyl, sec-butyl, allyl, methoxy, ethoxy, propoxy, isopropoxy, butoxy, acetamido, propionamido, iso-propionamido, fluoromethyl, difluoromethyl, trifluoromethyl, formyl, acetyl, propionyl, butyryl and oxime derivatives of the last four groups and O-methyl, ethyl, propyl, isopropyl and butyl ethers of said oximes.

In a specific embodiment Ar¹ is an unsubstituted 1,4-phenylene moiety Ar² is an optionally substituted heterocyclic moiety (as hereinbefore defined).

In a specific embodiment Ar¹ is unsubstituted and Ar² optionally substituted by one or two substituents independently selected from fluoro, chloro, bromo, iodo, hydroxy, nitro, methyl, ethyl, propyl, isopropyl, butyl, isobutyl, sec-butyl, allyl, methoxy, ethoxy, propoxy, isopropoxy, butoxy, acetamido, propionamido, iso-propionamido, fluoromethyl, difluoromethyl, trifluoromethyl, formyl, acetyl, propionyl, butyryl and oxime derivatives of the last four groups and O-methyl, ethyl, propyl, isopropyl and butyl ethers of said oximes.

In one embodiment of the present invention, R¹ and R² are both hydrogen; and Ar¹ and Ar² have any of the meanings defined above.

In a further embodiment of the present invention, R¹ is hydroxy; R² is hydrogen; and Ar¹ an Ar² have any of the meanings defined above.

In a further embodiment of the present invention, R¹ and R² are joined together so that CR¹⁻CR² is a double bond; and Ar¹ and Ar² have any of the meanings defined above.

In a particular aspect of the present invention there is provided a compound of formula I, or a pharmaceutically acceptable salt thereof, wherein:
R¹ is hydrogen or hydroxy;
R² is hydrogen; or
R¹ and R² are joined together so that CR¹⁻CR² is a double bond;
Ar¹ is a phenylene moiety and Ar² is a heterocyclic moiety selected from an aromatic 5-membered or 6-membered heterocyclic ring containing one, two or three heteroatoms selected from nitrogen, oxygen and sulphur; an aromatic 5-membered or 6-membered heterocyclic ring containing one, two or three heteroatoms selected from nitrogen, oxygen and sulphur which is fused to a benzene ring; and a bicyclic heterocycle which consists of a non-aromatic 5-membered or 6-membered heterocyclic ring containing (in addition to carbon atoms) one, two or three heteroatoms selected from nitrogen, oxygen and sulphur fused to a benzene ring;
and wherein one or both of Ar¹ and Ar² may optionally bear one or more substituents independently selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)dkylamino, di-[(1-6C)alkyl]amino N-(1-6C)alkylcarbmnoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno (1-6C)alkyl, (1-6C)alkanoylamino, ureido, N'-(1-6C)alkylureido, (1-6C)alkanoyl and oxime derivatives thereof and O-(1-6C)alkyl ethers of said oxime derivatives; provided that Ar² is not a 6-membered heteroaryl moiety containing one or two nitrogen atoms.

Particular, preferred and specific values are the appropriate values mentioned above.

Thus, in an embodiment of the present invention of particular interest there is provided a compound of formula I, or a pharmaceutically acceptable salt thereof wherein R¹ is hydroxy, R² is hydrogen, Ar¹ is a phenylene moiety, Ar² is a heterocyclic moiety selected from an aromatic 5-membered or 6-membered heterocyclic ring containing one, two or three heteroatoms selected from nitrogen, oxygen and sulphur; an aromatic 5-membered or 6-membered heterocyclic ring containing one, two or three heteroatoms selected from nitrogen, oxygen and sulphur which is fused to a benzene ring; and a bicyclic heterocycle which consists of a non-aromatic 5-membered or 6-membered heterocyclic ring containing (in addition to carbon atoms) one, two or three heteroatoms selected from nitrogen, oxygen and sulphur fused to a benzene ring;
and wherein one or both of Ar¹ and Ar² may optionally bear one or more substituents independently selected from halogeno, hydroxy, amino, nitro cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino, N-(1-6C)alkylcarbamoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl, (1-6C)alkanoylamino, ureido, N'-(1-6C)alkylureido, (1 -6C)alkanoyl and oxime derivatives thereof and O-(1-6C)alkyl ethers of said oxime derivatives; provided that Ar2 is not a 6-membered heteroaryl moiety containing one or two nitrogen atoms.

Particular, preferred and specific values are the appropriate values mentioned above.

In further embodiment of interest the present invention there is provided a compound of formula I, or a pharmaceutically-acceptable salt thereof wherein R¹ is hydroxy, R² is hydrogen, Ar¹ is a 1,4-phenylene moiety, Ar² is a 5- or 6- membered heteroaryl moiety containing one, two or three heteroatoms selected from nitrogen, oxygen and sulphur, which moiety may optionally be fused to a benzene ring; and wherein one or both of Ar¹ and Ar² may optionally bear one or more substituents independently selected from halogeno, hydroxy, amino, nitro cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino, N-(1-6C)alkylcarbamoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno-(1-6C)alkyl, (1-6C)alkanoylamino, ureido, N'-(1-6C)alkylureido, (1-6C)alkanoyl and oxime derivatives thereof and O-(1-6C)alkyl ethers of said oxime derivatives; provided that Ar2 is not a 6-membered heteroaryl moiety containing one or two nitrogen atoms.

Particular, preferred and specific values are the appropriate values mentioned above.

In a specific embodiment of the present invention there is provided a compound of formula I, or a pharmaceutically acceptable salt thereof, wherein; R¹ is hydroxy; R² is hydrogen; Ar¹ is 1,4-phenylene and Ar² is pyrrolyl, benzthiazolyl, oxadiazolyl or quinolyl, and Ar¹ and Ar² are optionally substituted as defined above.

Compounds of the invention which are of particular interest include the compounds described in the accompanying Examples (and their pharmaceutically-acceptable salts), and are hence provided as a further feature of the present invention. In particular the present invention provides a compound as described in Example 5,12 or 13 (or a pharmaceutically acceptable salts thereof).

A suitable pharmaceutically-acceptable salt of the present invention comprises an acid-addition salt derived from an inorganic or organic acid which provides a pharmaceutically-acceptable anion. Thus, examples of salts of the present invention include acid-addition salts with hydrochloric, hydrobromic, nitric, sulphuric, phosphoric, trifluoroacetic, citric, tartaric, succinic, maleic, fumaric or acetic acid. In addition, suitable pharmaceutically-acceptable salts include [where the compound of formula I is sufficiently acidic, for example where the compound of formula I bears an acidic substituent such as carboxy] those formed with a base which affords a pharmaceutically acceptable cation. Suitable bases include an alkali metal salt (such as a sodium or potassium salt), an alkaline earth metal salt (such as a calcium or magnesium salt), an ammonium salt or a salt with an organic base which affords a physiologically-acceptable cation such as a salt with methylamine, dimethylamine, triethylamine, piperidine or morpholine.

The compounds of the present invention may be obtained by standard procedures of organic chemistry already known to be applicable to the preparation of structurally analogous compounds. Such procedures for the preparation of the compounds of formula I, or pharmaceutically acceptable salts thereof, are provided as a further feature of the present invention and are illustrated by the following preferred processes in which the various generic radicals, for example, R¹, R², Ar¹ and Ar² have any of the meanings defined hereinbefore, and in which Ar¹ and Ar² may be unsubstituted or substituted as hereinbefore defined.

(a) For those compounds of formula I in which R¹ is hydroxy and R² is hydrogen, reacting a compound of formula Ar²-Ar¹-M in which M is a metal atom or a derivative thereof, with quinuclidin-3-one.

Suitable values for M include, for example, magnesium and lithium. In the case where M is magnesium it is conveniently present in the form of a derivative such as the group -MgX where X is a halogeno atom, such as iodo or bromo, so that the compound of formula Ar²-Ar¹-M is a "Grignard Reagent". The reaction is generally carried out in an inert solvent, such as dry diethyl ether or dry tetrahydrofuran, and at a temperature from about ambient temperature to the reflux temperature of the reaction mixture.

The compounds of formula Ar²-Ar¹-M may be prepared by reacting a compound of formula Ar²-Ar¹-hal in which "hal" is a halogeno atom such as iodo or bromo with the appropriate metal. For example in the case where M is the group -MgX, the compound of formula Ar²-Ar¹-hal may be reacted with magnesium turnings as is well known in the art. The desired Grignard Reagent may also be prepared by a transmetallation reaction, for example by reaction of a compound of formula Ar²-Ar¹-hal with a Grignard Reagent such as MeMgBr. Where M is a metal atom such as lithium, the compound of formula Ar²-Ar¹-M may be prepared by reaction of a compound of formula Ar²-Ar¹-hal with lithium in an inert solvent such as dry ether or tetrahydrofuran at a temperature below 0°C, such as at about -70°C. Alternatively, the compound of formula Ar²-Ar¹-hal may be treated with an alkyl lithium compound such as sec-butyl lithium.

The compounds of formula Ar²-Ar¹-hal may be prepared from compounds of formula H₂N-Ar¹-hal in which "hal" is a halogen atom such as iodo or bromo. The compound of formula H2N-Ar¹-hal is treated with sodium nitrite and concentrated hydrochloric acid with cooling as described in Example 1 part(a) to generate a diazonium salt which is then treated with the compound, Ar²_{,} in the presence of a base such as sodium hydroxide to give the compound of formula Ar²-Ar¹-hal. Positional isomers may be separated using conventional techniques such as column chromatography.

Alternatively, the compounds of formula Ar²-Ar¹-hal may be prepared by reaction of the appropriate compound of formula Ar²⁻X in which X is a leaving group such as halogen (for example, bromo) or trifluoromethanesulphonyloxy, with the appropriate compound of formula hal-Ar¹-B(L¹)L² in which "hal" is halogen such as bromo and L¹ and L2 are suitable ligands such as hydroxy. The reaction will be carried out in the presence of a catalyst such as tetrakis(triphenylphosphine)-palladium(-0). Suitable reaction conditions are those mentioned in (b) below. Similarly, compounds of formula Ar²-Ar¹-hal may be prepared by reaction of a compound of formula Ar²⁻B(L¹)L² with a compound of formula X-Ar¹-hal.

Compounds of formula Ar²-Ar¹-hal may also be prepared by other methods well known to those skilled in the art. For example, when Ar² is pyrrole, by reaction with a 2,5-diethoxytetrahydrofuran with a compound of formula hal-Ar¹⁻NH₂.

(b) Reacting a compound of formula II wherein L¹ and L² are suitable ligands with a compound of formula Ar²-X, in which X represents a leaving group, in the presence of a catalyst.

Suitable values for X include, for example, halogen such as bromo or iodo, and a trifluoromethanesulphonyloxy group. Suitable values for the ligands L¹ and L² present on the boron atom include groups independently selected from hydroxy, (1-4C)alkoxy (such as methoxy or ethoxy) and (1-6C)alkyl (such as methyl, ethyl, propyl or butyl). The groups L¹ and L² may, together with the boron atom to which they are attached, form a boroxin ring. The groups L¹ and L² may be joined together to define an-oxyalkyleneoxy- group so that L¹ and L² together with the boron atom define a cyclic borate ester group. A particularly suitable leaving group is the group -B(OH)₂.

Suitable catalysts include, for example, a catalyst selected from a palladium (0), palladium (II), nickel (0) and nickel (II) catalyst. Particular catalysts include, for example, tetrakis-(triphenylphosphine)nickel(0), bis(triphenylphosphine)nickel(II) chloride, nickel(II)chloride, palladium(II)chloride,
bis(triphenylphosphine)palladium(II)chloride, bis(triphenylphosphine)phenylpalladium iodide and tetrakis(triphenylphosphine)palladium(0). A radical initiator, for example, azo(bisisobutyronitrile) may also be present.

The process is generally performed in the presence of a suitable solvent or diluent, for example, a hydrocarbon, such as toluene or xylene, or an ether such as dioxan or tetrahydrofuran, and at a temperature in the range, for example, 20-150°C.

Compounds of formula II may be prepared by reaction of a compound of formula RO-BL¹(L²), such as a compound of formula B(OR)₃, wherein R is (1-6C)alkyl with a Grignard Reagent or aryllithium compound derived from a compound of formula IIIa in which X is halogen such as bromo in an analogous manner to the preparation of compounds of formula Ar²-B(L¹)L² mentioned below.

Alternatively, compounds of formula II in which R¹ is hydroxy and R² is hydrogen may be prepared by reaction of quinuclidin-3-one with a Grignard Reagent or aryllithium compound derived, using standard procedures such as those mentioned in (a) above, from a compound of formula hal-Ar¹-B(L¹)L² in which "hal" represents halogen such as bromo or iodo and L¹ and L² are as defined above, and in which they are preferably joined together so that L¹ and L² together to form an oxyalkyleneoxy group in which the alkylene moiety may bear one or more alkyl groups. The compounds of formula hal-Ar¹-B(L¹)L² in which R¹ is hydroxy and R² is hydrogen may be converted into those in which R¹ and R² are both hydrogen or R¹ and R² are joined together so that CR¹-CR² is a double bond using standard procedures such as those mentioned under (d), (e), (f) or (g) below.

(c) Reacting a compound of formula III in which X is a suitable leaving group with a compound of formula hal-Ar¹-B(L¹)L² in which L¹ and L² are suitable ligands in the presence of a catalyst.

Suitable values for X, L¹ and L², suitable catalysts and suitable reaction conditions are those mentioned under (b) above.

The compounds of formula hal-Ar¹-B(L¹)L² may be prepared by reaction of a boron compound of formula RO-BL¹(L²) in which L¹ and L² are alkyl or alkoxy groups as defined above. Thus, for example, a compound of formula B(OR)₃ wherein R is a (1-6C)alkyl group may be reacted with a Grignard Reagent or aryllithium compound derived, using standard procedures such as those mentioned in (a) above, from a compound of formula Ar²-hal wherein "hal" represents a halogeno atom such as bromo or iodo. The reaction is generally carried out in a solvent such as tetrahydrofuran or diethyl ether, or a mixture thereof, and at a temperature of -78°C to 25°C. The compounds of formula V wherein the ligands attached to boron are alkoxy may be converted to those in which the ligands are hydroxy by hydrolysis using standard techniques. The boroxin compounds may be prepared from the latter by dehydration using standard procedures.

The compounds of formula III in which R¹ is hydroxy and R² is hydrogen may be prepared by reaction of quinuclidin-3-one with a aryllithium compound derived, using procedures such as those analogous to those mentioned under (a) above, from a compounds of formula hal-Ar¹-X in which "hal" represents a halogen atom such as bromo or iodo and X is as defined above. The compounds of formula III in which R¹ is hydroxy and R² is hydrogen may be converted into compounds of formula III in which R¹ and R² are both hydrogen using standard procedures such as those mentioned in (d) or (g) below, and into compounds in which R¹ and R² are joined so that CR¹-CR² is a double bond using standard procedures such as those mentioned in (e) or (f) below.

(d) For those compounds of formula I in which R¹ and R² are both hydrogen, reducing a compound of formula I in which R¹ and R² are joined together so that -CR¹-CR² is a double bond.

The reduction may be carried out, for example, by catalytic hydrogenation, or by reaction with a suitable reducing agent. Suitable reaction conditions include, for example, catalytic hydrogenation using a catalyst which comprises a noble metal. Particular catalysts include palladium, platinum, nickel and supported catalysts such as Pd/C. The reduction is conveniently carried out in a solvent of, for example, an alcohol such as ethanol, and at (or near) ambient temperature and optionally under pressure.

Further suitable reaction conditions include, for example, reduction with a borane such as diborane. The reaction is generally carried out in an inert solvent of, for example, tetrahydrofuran or methyl t-butyl ether at, for example, a temperature of 0 to 60°C. It may be preferable to cool the reaction below ambient temperature (eg. to about 0°C) during the reduction. The borane generated may be hydrolysed by treatment with an organic acid such as acetic acid.

(e) For those compounds of formula I in which R¹ and R² are joined together so that CR¹-CR² is a double bond, dehydrating a compound of formula I in which R¹ is hydroxy.

The dehydration may be carried out using an acid such as sulphuric acid (for example, concentrated sulphuric acid), or p-toluene sulphonic acid. The reaction may conveniently be carried out with heating. For example, the reaction may be carried out using p-toluene sulphonic acid in a hydrocarbon solvent of, for example, toluene or xylene at ambient temperature to reflux, and preferably at reflux. The dehydration may also be carried out using trifluoroacetic acid in an inert solvent such as dichloromethane and at a temperature from ambient temperature to the reflux temperature of the reaction mixture.

(f) For compounds of formula I in which R¹ and R² are joined together so that CR¹-CR² is a double bond, treating a compound of formula IV in which X is a leaving group with a base.

Suitable values for X include, for example, halogeno such as chloro, bromo or iodo, or a sulphonyloxy group such as methanesulphonyloxy or toluenesulphonyloxy. Suitable bases include alkali metal hydroxides (such as potassium or sodium hydroxide), and alkali metal alkoxides (such as potassium t-butoxide or sodium ethoxide).

The reaction may conveniently be carried out in the presence of a solvent, preferably a polar organic solvent. Suitable solvents include, for example, an alcohol (such as ethanol), or an aprotic solvent such as dimethylformamide or N-methyl pyrrolidone. The reaction may be carried out at ambient temperature or at an elevated temperature such as at temperature between ambient and the reflux temperature of the reaction mixture.

The compounds of formula IV may be prepared, for example, from compounds of formula I in which R¹ is hydroxy and R² is hydrogen using methods well known in the art. For example, compounds of formula IV in which X is halogen may be prepared by reaction of formula I in which R¹ is hydroxy with the appropriate phosphorus halide (for example PC13, PBr₃ or PI₃) or, where X is chloro, by reaction with thionyl chloride. Compounds of formula IV in which X is methanesulphonyloxy or toluenesulphonyloxy may be prepared by reaction of the compound of formula I in which R¹ is hydroxy with mesyl chloride or tosyl chloride respectively.

(g) For those compounds of formula I in which R¹ and R² are both hydrogen, by dehydroxylation of a compound of formula I in which R¹ is hydroxy.

The reaction may be carried out by catalytic hydrogenation. Suitable reaction conditions include those mentioned under (d) above. The reaction may also be carried out using, for example, trifluoroacetic acid and Et₃SiH, conveniently at a temperature between ambient temperature and the reflux temperature of the reaction mixture (eg. at about 50°C).

It will be appreciated that in some of the reactions mentioned herein it may be necessary/desirable to protect any sensitive groups in the compounds. The instances where protection is necessary or desirable and suitable methods for protection are known to those skilled in the art. Thus, if reactants include groups such as amino, carboxy or hydroxy it may be desirable to protect the group in some of the reactions mentioned herein. Suitable protecting groups for hydroxy include, for example, silyl groups such as trimethylsilyl or t-butyldimethylsilyl, tetrahydropyranyl and esterifying groups such as a methyl or ethyl ester; and for amino groups include benzyloxycarbonyl and t-butoxycarbonyl. Carboxy groups may be protected in a reduced form such as in the form of the corresponding protected alcohol, which may be subsequently oxidised to give the carboxy group. The protecting groups may be removed at any convenient stage in the synthesis using conventional techniques well known in the chemical art.

It will also be appreciated that the preferred process for preparing a particular compound of formula I will depend upon the nature of the various radicals. Similarly, the preferred choice of reagent will depend upon the nature of the various radicals present. For example, when it is required to reduce a particular compound the reducing agent will generally be selected to be one which does not interfere with other groupings present.

It will also be appreciated that certain of the various optional substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acylhalide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulphinyl or alkylsulphonyl.

When a pharmaceutically-acceptable salt of a compound of the formula I is required, it may be obtained, for example, by reaction of said compound with the appropriate acid (which affords a physiologically acceptable anion), or with the appropriate base (which affords a physiologically acceptable cation), or by any other conventional salt formation procedure.

As mentioned previously, the compounds of the formula I (and their pharmaceutically-acceptable salts) are inhibitors of the enzyme squalene synthase. Thus the compounds of the present invention are capable of inhibiting cholesterol biosynthesis by inhibition of de novo squalene production.

The beneficial pharmacological properties of the compounds of the present invention may be demonstrated using one or more of the following techniques.

### (a) Inhibition of Squalene synthase

In this test, the ability of a compound to prevent the formation of squalene from a radioactive substrate (tritiated farnesyl pyrophosphate) is assessed.

The test compound is incubated at a concentration of 25 micromolar in 200µl of a buffered solution containing potassium phosphate (50mM), MgCl₂ (4.95mM), KF (9.9mM), NADPH (0.9mM) and rat liver microsomal protein (20µg). Rat liver microsomes are prepared by the method described in published European Patent Application No. 324,421 and stored in liquid nitrogen prior to assay. Assay vials are kept at 37°C throughout the incubation.

The reaction is started with the addition of the substrate (1-(³H]-farnesyl pyrophosphate), final concentration 20µM, and stopped after 15 minutes reaction time with the addition of 50µl of 4% KOH. The reaction products are separated from unreacted substrate after application to a C-18 octadecyl 1ccBond column (Analytichem Int product No. 617101). An aqueous fraction is eluted with 250µl of 0.1M KOH. Squalene is then eluted with 1.0 ml 10% ethylacetate in hexane and radioactivity determined. The difference in radioactivity in the presence and absence of the test compound is used to determine the level of inhibition. If the test compound inhibits at greater than about 70% at 25 micromolar, it is generally re-tested at 25 and 2.5 micromolar. The IC₅₀ (concentration which results in a 50% inhibition of squalene production), of the test compound can be determined by testing the compound at several, for example five, concentrations predicted from the two concentration results. The IC₅₀ can then be determined from a plot of percentage inhibition against concentration of test compound.

In general, compounds of formula I show significant inhibition in the above test at a concentration in the range of about 0.001 to 25µM.

By way of illustration of the squalene synthase inhibitory properties of the compound of formula I, described in Example 3 below gave an inhibition of about 71% at a concentration of 2.5µM.

### (b) Acute rat cholesterol synthesis assay.

This is an acute in vivo test in the rat to measure de novo hepatic cholesterol synthesis from exogenously administered ¹⁴C-acetate.

Female rats (35 - 55 g) are housed in reverse lighting conditions (red light from 0200h - 1400h) for a period of about 2 weeks prior to test. Animals are allowed free access to chow and drinking water throughout this period. At test, animals should weigh 125 - 150 g. Test compounds may be administered by oral gavage, dissolved or suspended in 0.5% polysorbate, or by ip or iv dosing. Control animals receive vehicle alone. After 1 hour the rats are injected ip with 25µCi [2-¹⁴C]-acetate (NEN DUPONT. specific activity, 45-60mCi/mmol NEC-085H, or AMERSHAM specific activity, 50-60mCi/mmol CFA 14) in a volume of 0.25 ml saline (100µCi/ml). After a further hour, rats are terminally anaesthetised with halothane and a blood sample obtained from the abdominal vena cava.

1ml of plasma is lyophilised and then saponified in 2ml ethanolic KOH (1 part 33% KOH, 9 parts ethanol) at 75°C for 2 hours. After addition of an equal quantity of water, non-saponifiable lipids are extracted with two 5ml volumes of hexane. The hexane extracts are evaporated to dryness and the residues dissolved in ethanol to determine cholesterol specific radioactivity. ED₅₀ values can be determined in the standard way.

In general, compounds of formula I show activity in the range of about 0.1 to 100 mg/kg.

By way of illustration, the compound of formula I described in Example 3 gave a 74% reduction in the rate of cholesterol biosynthesis and an ED₅₀ of about 14 mg/kg; the compound described in Example 12 gave an ED₅₀ of about 3.8mg/kg and the compoud described in Example 6 gave an ED₅₀ of about 4.5mg/kg.

In an alternative in vivo test, de novo hepatic cholesterol synthesis from exogenously administered ³H-mevalonolactone is measured. The above procedure is used but with ³H-mevalonolactone (2.6µCi) administered in place of ¹⁴C-acetate and the test compound is generally administered as a solution or suspension in 10% dimethylsulphoxide in 0.5% hydroxypropylmethylcellulose.

No overt toxicity was detected when compounds of the formula I were administered at several multiples of their minimum inhibitory dose or concentration.

As mentioned above, the compounds of the present invention are squalene synthase inhibitors and hence possess the property of inhibiting cholesterol biosynthesis. Thus the compounds of the present invention will be useful in treating diseases or medical conditions in which an inhibition of squalene synthase is desirable, for example those in which a lowering of the level of cholesterol is blood plasma is desirable. In particular, the compounds of the present invention will be useful in treating hypercholesterolemia and/or ischaemic diseases associated with atheromatous vascular degeneration such as atherosclerosis. The compounds of the present invention will also be useful in treating fungal infections.

Thus according to a further feature of the present invention there is provided a method of inhibiting squalene synthase in a warm-blooded animals (such as man) requiring such treatment, which method comprises administering to said animal an effective amount of a compound of formula I, or a pharmaceutically-acceptable salt thereof, wherein:
R¹ is hydrogen or hydroxy;
R² is hydrogen; or
R¹ and R² are joined together so that CR¹-CR² is a double bond;
Ar¹ is a phenylene moiety and Ar² is a heterocyclic moiety selected from an aromatic 5-membered or 6-membered heterocyclic ring containing one, two or three heteroatoms selected from nitrogen, oxygen and sulphur; an aromatic 5-membered or 6-membered heterocyclic ring containing one, two or three heteroatoms selected from nitrogen, oxygen and sulphur which is fused to a benzene ring; and a bicyclic heterocycle which consists of a non-aromatic 5-membered or 6-membered heterocyclic ring containing (in addition to carbon atoms) one, two or three heteroatoms selected from nitrogen, oxygen and sulphur fused to a benzene ring;
and wherein one or both of Ar¹ and Ar² may optionally bear one or more substituents independently selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino N-(1-6C)alkylcarbamoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno (1-6C)alkyl, (1-6C)alkanoylamino, ureido, N'-(1-6C)alkylureido, (1-6C)alkanoyl and oxime derivatives thereof and 0-(1-6C)alkyl ethers of said oxime derivatives; provided that Ar² is not a 6-membered heteroaryl moiety containing one or two nitrogen atoms; and that when R¹ and R², are both hydrogen, Ar¹ is not an oxadiazole moiety.

In particular, the present invention provides a method of inhibiting cholesterol biosynthesis, and more particularly to a method of treating hypercholesterolemia and atheromatous vascular degeneration (such as atherosclerosis).

Thus the present invention also provides the use of a compound of formula I (as herein defined), or a pharmaceutically-acceptable salt thereof, for the manufacture of a medicament for treating diseases or medical conditions in which a lowering of the level of cholesterol in blood plasma is desirable (such as hypercholesterolemia and atherosclerosis).

When used in the treatment of diseases and medical conditions in which an inhibition of cholesterol biosynthesis is desired, for example in the treatment of hypercholesterolemia or atherosclerosis, it is envisaged that a compound of formula I (or a pharmaceutically acceptable salt thereof) will be administered orally, intravenously, or by some other medically acceptable route so that a dose in the general range of, for example, 0.01 to 50 mg per kg body weight is received. However it will be understood that the precise dose administered will necessarily vary according to the nature and severity of the disease, the age and sex of the patient being treated and the route of administration.

In general, the compounds of formula I (or a pharmaceutically-acceptable salt thereof) will usually be administered in the form of a pharmaceutical composition, that is together with a pharmaceutically acceptable diluent or carrier, and such a composition is provided as a further feature of the present invention.

A pharmaceutical composition of the present invention may be in a variety of dosage forms. For example, it may be in the form of tablets, capsules, solutions or suspensions for oral administration, in the form of a suppository for rectal administration; in the form of a sterile solution or suspension for parenteral administration such as by intravenous or intramuscular injection.

A composition may be obtained by conventional procedures using pharmaceutically acceptable diluents and carriers well known in the art. Tablets and capsules for oral administration may conveniently be formed with a coating, such as an enteric coating (for example, one based on cellulose acetate phthalate), to minimise dissolution of the active ingredient of formula I (or a pharmaceutically-acceptable salt thereof) in the stomach or to mask unpleasant taste.

The compounds of the present invention may, if desired, be administered together with (or sequentially to) one or more other pharmacological agents known to be useful in the treatment of cardiovascular disease, for example, together with agents such as HMG-CoA reductase inhibitors, bile acid sequestrants, other hypocholesterolaemic agents such as fibrates, for example gemfibrozil, and drugs for the treatment of coronary heart disease. As a further example, the compounds of the present invention may, if desired, be administered together with (or sequentially to) an angiotensin converting enzyme (ACE) inhibitor, such as captopril, lisinopril, zofenopril or enalapril.

Compounds which inhibit squalene synthase have also found utility as antifungal agents. Thus the present invnetion also provides a method of treating fungal infections comprising adimistering a compound of formula I, or a non-toxic salt thereof, to an organism in need of such treatment.

In particular, the present invention also provides a method of treating fungal infections which comprises administration to an a warm blooded animal, such as man, in need of such treatment an effective amount of a compound of formula I, or a pharmaceutically acceptable salt thereof.

When used as anti-fungal agents the compounds may be formulated in a variety of ways, the nature of such formulation depending on whether the use is for controlling pathogens infecting mammals such as man, or in agriculture such as in soil or plants, or some other object. For medical applications, the compounds of the present invention may, in addition to the formulations mentioned above, be adapted for topical administration and such a composition is provided as a further feature of the present invention. Such compositions may be in a variety of forms, for example creams or lotions.

The invention will now be illustrated by the following Examples in which, unless otherwise stated:-
(i) evaporations were carried out by rotary evaporation in vacuo;
(ii) operations were carried out at room temperature, that is in the range 18-26°C;
(iii) flash column chromatography or medium pressure liquid chromatography (MPLC) was performed on silica gel (Merck Kieselgel Art.9385, obtained from E Merck, Darmstadt, Germany);
(iv) yields are given for illustration only and are not necessarily the maximum attainable by diligent process development;
(v) proton NMR spectra were normally determined at 200 MHz using tetramethylsilane (TMS) as a internal standard, and are expressed as chemical shifts (delta values) in parts per million relative to TMS using conventional abbreviations for designation of major peaks: s, singlet; m, multiplet; t, triplet; br, broad; d, doublet;
(vi) all end-products were characterised by microanalysis, NMR and/or mass spectroscopy; and
(vii) conventional abbreviations are used for individual radicals and recrystallisation solvents, for example, Me = methyl, Et = ethyl, Pr = Propyl, Pri = isopropyl, Bu = butyl, Bui = isobutyl, Ph = phenyl; EtOAc = ethyl acetate, Et₂O = ether, MeCN = acetonitrile, MeOH = methanol, EtOH = ethanol, PrⁱOH = 2-propanol, H₂O = water.

### EXAMPLE 1

A solution of sec-butyllithium in cyclohexane (29 ml, 1.3M) was added dropwise with stirring to a solution of 1-(4-bromophenyl)-2,5-dimethylpyrrole (9.06 g) in dry tetrahydrofuran (120 ml) under an atmosphere of argon at -78°C. The mixture was stirred for 5 minutes and a solution of quinuclidin-3-one (4.3 g) in dry tetrahydrofuran (30 ml) added over a period of 7 minutes. Stirring was continued at -78°C for 30 minutes and the mixture was then allowed to reach room temperature over a period of 2 hours. Water (200 ml) was then added to the reaction mixture whilst keeping the reaction temperature below 10°C. The mixture was extracted with ethyl acetate, the ethyl acetate phase separated, dried (Na₂SO₄) and evaporated to yield a residue which was purified by medium pressure column chromatography on alumina (ICN Alumna N 32-63), eluting with ethyl acetate. The product was further purified by crystallisation from ethyl acetate to give 3-[4-(2,5-dimethylpyrrol-1-yl]phenyl)-quinuclidin-3-ol (5.0 g) as a colourless solid, m.p. 215-216°C; microanalysis, found: C, 76.9; H, 8.2; N, 9.2%; C₁₉H₂₄N₂O requires: C, 77.0; H, 8.2; N, 9.4%; NMR ([CD₃]₂SO/CD₃COOD): 1.5-1.6(1H, m), 1.7-1.9(2H, m), 2.4(2H, s), 2.55(6H, s), 3.1-3.3(4H, m), 3.4-3.5(1H, d), 3.85-3.95(1H, d), 5.8(2H, s), 7.3-2H, d) and 7.7(2H, d): m/Z 297 (M+H).

The preparation of 1-(4-bromophenyl)-2,5-dimethylpyrrole is described in, for example, J.Chem.Soc. Perkin 1, (1984), 2801.

### EXAMPLE 2

Using a similar procedure to that in Example 1 but using 1-(4-bromophenyl)pyrrole as starting material (in place of 1-(4-bromophenyl)-2,5-dimethylpyrrole) there was obtained 3-[4-(pyrrol-1-yl)phenyl]quinuclidin-3-ol (10% yield), m.p. 176-177°C, microanalysis, found: C, 75.8; H, 7.6; N, 9.9%; C₁₇H₂₀N₂O requires: C, 76.1; H, 7.5; N, 10.4%; NMR ([CD₃]₂SO), 1.2-1.5(3H, m), 1.9-2.0(1H, s), 2.1-2.2(1H, m), 2.6-2.8(4H, m), 2.8-2.9(1H, d), 3.3-3.4(1H, d of d), 5.1(1H, s), 6.25(2H, d oft), 7.3(2H, d of d) and 7.5-7.6(4H, m): m/Z 269 (M+H).

The preparation of 1-(4-bromophenyl)pyrrole is described in, for example, Australian Journal of Chemistry, (1966), 19, 289.

### EXAMPLE 3

A solution of tert-butyl lithium in pentane (2.4 ml, 1.7M) was added dropwise with stirring to a solution of 2-(4-bromophenyl)quinoline (566 mg) in dry tetrahydrofuran (30 ml) under an atmosphere of argon at -78°C. The reaction mixture was stirred for 0.5 hours and a solution of quinuclidin-3-one (225 mg) in tetrahydrofuran (8 ml) was then added dropwise over a period of 10 minutes. Stirring was continued at -78°C for 2.5 hours and the mixture was then allowed to reach 0°C over a period of 2 hours. Water (100 ml) and 2M aqueous sodium hydroxide solution (2 ml) were added. The mixture was extracted with ethyl acetate, the ethyl acetate phase separated, dried (Na₂SO₄) and evaporated to give a colourless solid which was purified by crystallisation from propan-2-ol to give 3-[4-(2-quinolyl)phenyl)quinuclidin-3-ol (286 mg) as a colourless solid, m.p. 228-230°C; microanalysis, found: C, 79.6; H, 69.0; N, 8.10%; C₂₂H₂₂N₂O requires: C, 80.0; H, 6.71; N, 8.48%; NMR [(CD₃)₂SO]: 1.22-1.52(3H, m), 1.95-2.02(1H, m), 2.10-2.25(1H, m), 2.60-2.94(5H, m), 3.44(1H, d), 5.21(1H, s), 7.50-7.86(4H, m), 7.95-8.35(3H, m), 8.24(2H, d) and 8.44(1H, d): m/Z 331 (M+H).

The preparation of 2-(4-bromophenyl)quinoline is described in, for example, CA 72, 31573e and J.Inst.Chem., Calcutta, (1969), 41, 138-141.

### EXAMPLE 4

A solution of sec-butylithium in cyclohexane (5.0 ml, 1.3M) was added dropwise with stirring to a solution of 2-(4-bromophenyl)-benzothiaz-ole (1.60 g) in tetrahydrofuran (25 ml) under an atmosphere of argon at -78°C. The reaction mixture was stirred for 15 minutes and a solution of quinuclidine-3-one (625 mg) in tetrahydrofuran (10 ml) was then added over a period of 6 minutes. Stirring was continued at -78°C for 2 hours and the mixture was then allowed to reach room temperature over a period of 16 hours. 1M Aqueous hydrochloric acid solution (100 ml) was added whilst keeping the reaction temperature below 10°C. The aqueous phase was washed with ethyl acetate (1 x 50 ml) before the addition of excess sodium hydroxide solution (density 1.35 g/cm³) to pH14. The mixture was extracted with ethyl acetate, the ethyl acetate phase separated, dried (Na₂SO₄) and evaporated to give a colourless solid which crystallised from ethyl acetate to give 3-[4-(benzothiazol-2-yl)-phenyl)quinuclidin-3-ol (260 mg), m.p. 215.5-216°C; microanalysis, found: C, 71.3; H, 6.0; N, 8.10%; C₂₀H₂₀N₂OS requires: C, 71.4; H, 5.99; N, 8.33%; NMR ([CD₃]₂SO): 1.20-1.56(3H, m), 1.98(1H, bs), 2.08-2.28(1H, m), 2.60-3.00(5H, m), 3.41(1H, d), 5.31(1H, s), 7.40-7.60(2H, m), 7.71(2H, d), 8.05(3H, d) and 8.13(1H, d of d); m/Z 337 (M+H).

The preparation of 2-(4-bromophenyl)-benzothiaz-ole is described in, for example, Cancer Res., (1968), 28,2539-2544.

### EXAMPLE 5

A solution of sec-butyllithium in cyclohexane (13 ml, 1.3M) was added dropwise with stirring to a solution of 5-(4-iodophenyl)-3-methyl1,2,4-oxadiazole (505 mg) in dry tetrahydrofuran (10 ml) under an atmosphere of argon at-78°C. The mixture was stirred for 10 minutes and a solution of quinuclidin-3-one (200 mg) in dry tetrahydrofuran (5 ml) was then added over a period of 6 minutes. Stirring was continued at -78°C for 3 hours and the mixture was then allowed to reach room temperature over a period of 16 hours. The mixture was added to ice and extracted with ethyl acetate, the ethyl acetate phase separated, dried (Na₂SO₄) and evaporated to give a residue which was crystallised from ethyl acetate to give 3-[4-(3-methyl-1,2,4-oxadiazol-5-yl)phenyl)-quinuclidin-3-ol (58 mg) as a colourless solid, m.p. 213-214°C; microanalysis, found: C, 64.00; H, 6.40; N, 13.3%; C₁₆H₁₉N₃O₂1.0H₂O requires: C, 63.6; H, 6.80; N, 13.7%; NMR ([CD₃]₂SO): 1.20-1.50(3H, m), 1.97(1H, bs), 2.02-2.22(1H, m), 2.41(3H, s), 2.60-3.00(5H, m), 3.41(1H, d), 5.34(1H, s), 7.75(2H, d) and 8.04(2H, d); m/Z 286 (M+H).

The starting 5-(4-iodophenyl)-3-methyl-1,2,4-oxadiazole was prepared by the following method:
Acetamide oxime hydrochloride (1.5 g) was added portionwise to sodium hydride (1.18g; 60% dispersion in oil) in dry tetrahydrofuran (40 ml) at 0-5° under an atmosphere of argon. 4_ molecular sieves (1 g) were added.
After 1 hour, a solution of ethyl-4-iodo benzoate (3.73 g) in tetrahydrofuran (10 ml) was added. The reaction mixture was heated for 3 hours at 60-65°. The reaction mixture was cooled to 0°C and water (50 ml) added. The mixture was extracted with ethyl acetate. The ethyl acetate phase was washed with saturated brine solution, dried (MgSO₄) and evaporated. The residue was purified by flash column chromatography on silica gel (Merck Art no 9385) using a 7:3 (v/v) mixture of hexane and ethyl acetate as eluent to give 5-(4-iodophenyl)-3-methyl-1,2,4oxadiazole (1.12 g) as a colourless solid; microanalysis: C, 38.2; H, 2.5; N, 9.5%; C₉H₇IN₂O requires C, 37.8; H, 2.47; N, 9.8%; NMR ([CD₃]₂SO): 2.41(3H, s), 7.77-7.90(2H, d) and 7.95-8.08(2H, d); m/Z 287 (M+H).

### EXAMPLE 6

In a similar manner to Example 4, but using 2-(4-bromophenyl)benzoxazole as starting material in place of 2-(4-bromophenyl)benzothiazole, there was obtained 3-[4- (benzoxazol-2-yl)phenyl]quinuclidin-3-ol as a solid, m.p. 255.5-256°C; microanalysis, found: C, 74.6; H, 6.20; N, 8.50%; C₂₀H₂₀N₂O₂ requires: C, 75.0; H, 6.30; N, 8.74; NMR([CD₃]₂SO): 1.40(3H,m), 2.00(1H,m), 2.81(1H,m), 2.60-3.00(5H,m), 3.45(1H,d), 5.35(1H,s), 7.45(2H,m), 7.80(4H,m) and 8.18(2H,d); m/z 321 (M+H).

The 2-(4-bromophenyl)benzoxazole used as starting material was prepared as follows.

Thionyl chloride (7.3ml) was added dropwise to a stirred suspension of 4-bromobenzoic acid (17.6g) in N-methylpyrrolidinone (50ml) at 10°C over a period of 20 minutes under an atmosphere of argon. The mixture was stirred at ambient temperature for 0.5 hours. 2-aminophenol (9.1g) was added. The mixture was then heated at 105°C for 0.5 hours and then at 145°C for 3 hours. The reaction mixture was cooled to 40° and then poured into a vigorously stirred mixture of ice (50g), water(50ml) and 40% sodium hydroxide solution (25ml). The mixture was stirred for 0.5 hours. The solid precipitate was collected by filtration and washed with water. The solid was stirred in ethyl acetate (300ml) and the mixture filtered. The filtrate was evaporated to give a solid which was purified by chromatography on silica gel (Merck Art 7734) using a 4:1 (v/v) mixture of hexane/ethyl acetate as eluent to give 2-(4-bromophenyl)benzoxazole (11.5g) as a solid, m.p. 157.5°C; NMR (CDCl₃): 7.38(2H,m), 7.57(1H,m), 7.67(2H,m), 7.76(1H,m) and 8.12(2H,m); m/z 274 (M+H).

### EXAMPLE 7

In a similar manner to Example 4, but using 2-(4-bromophenyl)- 5-methyloxazole in place of 2-(4-bromophenylbenzothiazole, there was obtained 3-[4-(5-methyloxazol-2-yl)phenyl]quinuclidin-3-ol as a solid, m.p 189-190°C; microanalysis, found: C, 71.5; H, 7.30; N, 9.60%; C₁₇H₂ON₂O₂ requires: C, 71.8; H, 7.09; N, 9.85%; NMR([CD₃]₂SO): 1.18-1.50(3H,m), 1.92(1H,m), 2.05-2.23(1H,m), 2.38(3H,s), 2.60-2.95(5H,m), 3.36(1H,d), 5.21(1H,s), 6.96(1H,d), 7.65(2H,d) and 7.91(2H,d); m/z 285 (M+H).

The 2-(4-bromophenyl)-5-methyl oxazole used as starting material was prepared as follows.

A solution of propargylamine (3.55g) in ethyl acetate (25ml) was added to a stirred mixture of 4-bromobenzoyl chloride (14.2g) and sodium carbonate (10.6g) in ethyl acetate (100ml) over a period of 0.25 hours at 25°C. The mixture was heated at reflux for 1 hour. The mixture was cooled to ambient temperature and water (200ml) and ethyl acetate (100ml) were added. The ethyl acetate phase was separated, washed with saturated sodium bicarbonate solution, dried (MgSO₄) and evaporated. The residue was crystallised from ethyl acetate to give 4-bromobenzoyl-N-propargylamide (8.9g) as a solid, m.p. 281.8°; NMR([CD₃]₂SO): 3.10(1H,m), 4.05(2H,m), 7.69(2H,m), 7.80(2H,m) and 9.00(1H,m); m/z 238 (M+H).

Mercuric acetate (40mg) was added to a mixture of 4-bromobenzoylpropargyl amide (3.57g) in acetic acid (30ml) and the mixture heated at reflux for 1.25 hours. The mixture was cooled to ambient temperature and the acetic acid was removed by evaporation. The residue was purified by chromatography on silica gel (Merck Art No 7734) using a 7:3 (v/v) mixture of hexane/ethyl acetate as eluent to give 2-(4-bromophenyl)-5-methyl-oxazole (2.75g) as a pale yellow solid, m.p. 66.8°C; NMR(CDCl₃): 2.40(3H,s), 6.81(1H,d), 7.52(2H,m) and 7.82(2H,m); m/z 238(M+H).

### EXAMPLE 8

In a similar manner to Example 4 but using N'-methyl-2-(4-bromophenyl)benzimidazole as starting material in place of 2-(4-bromophenyl)benzothiazole, there was obtained 3-[4-(N'-methylbenzimidazol-2-yl)phenyl]quinuclidin-3-ol as a solid, m.p. 243-5°C; microanalysis, found: C, 73.50; H, 6.90; N, 11.9%; C₂₁H₂₃N₃0 0.5 H₂0 requires: C, 73.70; H, 7.0; N, 12.2%; NMR([CD₃]₂SO): 1.25-1.58(3H,m), 2.02(1H,bs), 2.10-2.25(1H,m), 2.40-3.00(5H,m), 3.45(1H,d), 3.88(3H,s), 5.30(1H,s), 7.18-7.32(2H,m), 7.52-7.75(4H,m) and 7.82(2H,d); m/z 334 (M+H).

The N'-methyl-2-(4-bromophenyl)benzimidazole used as starting material was prepared as follows.

A solution of 4-bromobenzaldehyde (1.85g) in ethanol (5ml) was added to a solution of 2-N-methylaminoaniline (1.22g) in ethanol. The reaction mixture was stirred for 2 hours at room temperature. Nitrobenzene (6ml) was added and the reaction mixture was heated to 210°C (ethanol was removed during this period) and then heated at 210°C for 2 minutes. The reaction mixture was cooled to ambient temperature and ethyl acetate (50ml) and 2N aqueous hydrochloric acid (100ml) were added. N'-methyl-2-(4-bromophenyl)benzimidazole was precipiated as its hydrochloride salt and was collected by filtration. The crude hydrochloride salt was suspended in water (25ml) and basified with 2M aqueous sodium hydroxide solution. This aqueous solution was extracted with ethyl acetate. The ethyl actetate phase was separated, washed with brine (2 x 25ml), dried (MgSO₄) and evaporated. The residue was purified by medium pressure chromatography on silica gel using a 1:1 (v/v) mixture of hexane/ethyl acetate as eluent to give N'-methyl-2-(4-bromophenyl)benzimidazole (1.45g) as a solid, m.p. 112-114°C; microanalysis, found: C, 58.30; H, 3.90; N, 10.00%; C₁₄H₁₁N₂Br requires: C, 58.60; H, 3.86; N, 9.76%; NMR(CDCl₃): 3.85(3H,s), 7.25-7.42(3H,m), 7.65(4H,s) and 7.75-7.86(1H,m).

### EXAMPLE 9

Saturated sodium hydrogen carbonate solution (10ml) was added to a stirred solution of 3-(4-bromophenyl)-3-hydroxyquinuclidine (490mg), thiophene-2-boronic acid (320mg) and tetrakis triphenylphosphine palladium [O] (20mg) in dimethoxyethane (25ml) at ambient temperature and under an atmosphere of argon. The mixture was heated at reflux for 1.5 hours and then cooled to ambient temperature. Water (100ml) was added and the mixture extracted with ethyl acetate. The ethyl acetate phase was separated and extracted with 2M aqueous hydrochloric acid. The aqueous acid extract was basified to pH12 with 8M aqueous sodium hydroxide solution and then extracted with ethyl acetate. The ethyl acetate phase was washed with saturated brine, dried (MgSO₄) and evaporated. The residue was recrystallised from ethyl acetate to give 3-(4-(thien-2-yl)phenyl]quinuclidin-3-ol (301mg) as a grey solid, m.p. 165-8°C; microanalysis, found: C, 69.7; H, 6.70; N, 4.70%, C₁₇H₁₉NOS. 0.5H₂0 requires: C, 69.6; H, 6.80, N, 4.70%; NMR([CD₃]₂SO): 1.20-1.50(3H,m), 1.92(1H,m), 2.05-2.23(1H,m), 2.60-2.97(5H,m), 3.32(1H,d), 5.22(1H,s), 7.08-7.15(1H,m) and 7.42-7.65(6H,m); m/z 286(M+H).

The boronic acid used as starting material was prepared as follows.

A solution of borane in tetrahydrofuran (40ml, 1M) was added at 0°C, with stirring, to magnesium turnings (288mg) over a period of 0.33 hours whilst under an atmosphere of argon. 2-Bromothiophene (1.63g) was then added to the reaction mixture over a period of 0.25 hours and the resulting mixture stirred at room temperature for 16 hours. The mixture was added slowly, with stirring, to ice-water (250ml). Aqueous 2M hydrochloric acid was added to give a pH 1 solution. The aqueous mixture was extracted with diethyl ether, the ether phase was separated, washed with saturated brine, dried (MgSO₄) and evaporated. There was thus obtained thiophene-2-boronic acid (710mg) as a colourless solid; m/z 129 (M+H).

### EXAMPLE 10

A solution of sec-butyllithium in cyclohexane (7.5ml, 1.3M) was added dropwise over a period of 10 minutes to a solution of 4-bromophenylboronic acid [N-methyl-O;O'-diethanolamine]ester (1.42g) in dry tetrahydrofuran (25ml) under an atmosphere of argon at -100°C. The mixture was stirred for 30 minutes and a solution of quinuclidin-3-one (0.625g) in dry tetrahydrofuran (10ml) was then added over a period of 10 minutes whilst maintaining the temperature at -100°C. The reaction mixture was stirred for 1 hour at -100°C, allowed to warm to ambient temperature and then stirred at ambient temperature for a further 2 hours. An aqueous solution of ammonium chloride (0.55g) in water (5ml) was added and the mixture stirred for 0.5 hours. The tetrahydrofuran was then removed by evaporation. Toluene (20ml) and saturated sodium carbonate solution (10ml) were added to the residue. A solution of 2-bromothiazole (870mg) in absolute alcohol (10ml) was added to the mixture at 25°C whilst under an atmosphere of argon. Tetrakis triphenylphosphine palladium [O] (100mg) was added and the mixture heated at reflux for 4 hours. After cooling to ambient temperature, ice and 2M aqueous hydrochloric acid were added and the mixture washed with ethyl acetate. The aqueous phase was basified with 10M aqueous sodium hydroxide solution and then extracted with dichloromethane. The dichloromethane phase was separated, washed with saturated brine solution, dried (MgSO₄) and evaporated. The residue was triturated with ethyl acetate to give 3-[4-(thiazol-2-yl)phenyl]quinuclidin-3-ol (120mg) as a solid, m.p. 218-219°C; microanalysis, found: C, 65.0; H, 6.40; N, 9.40%, C₁₆H₁₈NO₂S 0.5 H₂0 requires: C, 65.0; H, 6.40; N, 9.50%; NMR([CD₃]₂SO): 1.20-1.52(3H,m), 1.93(1H,m), 2.02-2.22(1H,m), 2.53-2.95(5H,m), 3.40(1H,d), 5.22(1H,s), 7.62(2H,d), 7.75(1H,d) and 7.90(3H,m); m/z 289(M+H).

### EXAMPLE 11

Using a similar procedure to that in Example 1, but using 4-(4-bromophenyl)-1,2-methylenedioxybenzene as starting material (in place of 1-(4-bromophenyl)-2,5-dimethylpyrrole) there was obtained 3-[4-(3,4-methylenedioxyphenyl)phenyl]quinuclidin-3-ol (18% yield) as a solid, m.p. 167-169°C; microanlaysis, found: C, 74.0; H, 6.6; N, 4.1%; C₂₀H₂₁NO₃ requies: C, 74.3; H, 6.6; N, 4.3%; NMR:(CD₃]₂SO/CD₃COOD): 1.4-1.8(3H,m), 2.3(2.5(2H,m), 3.1-3.3(4H,m), 3.4-3.9(2H,d of d), 6.1(2H,s), 7.0(1H,d), 7.1-7.2(1H, d of d), 7.2-7.3(lH,d), and 7.6(4H,s); m/z 324 (M+H).

The 4-(4-bromophenyl-1,2-methylene dioxybenzene used as starting material was prepared as follows.

A 2M aqueous solution of sodium carbonate (12ml) was added to a stirred mixture of 4-bromo-1,2-methylenedioxybenzene (2.01g), 4-bromophenylboronic acid and tetrakis triphenylphosphine palladium (175mg) in toluene (60ml) under an argon atmosphere. The reaction mixture was heated at 70°C for 4 hours and then cooled to ambient temperature. The mixture was filtered and the filtrate was extracted with ethyl acetate. The ethyl acetate phase was separated, dried (MgSO₄) and evaporated.

The residue was purified by medium pressure column chromatography on silica gel using 5% ethyl acetate/hexane as eluent to give, after crystallisation from hexane, 4-(4-bromophenyl) 1,2-methylenedioxybenzene (385mg) as a colourless solid, m.p. 97-99°C; microanalysis, found: C, 56.7; H, 3.3%; C₁₃H₉Br0₂ requires: C, 56.3; H, 3.3%; NMR:(CDCl₃): 6.0(2H,s), 6.8-6.9(lH,d), 7.0(2H,m) and 7.3-7.5(4H, d of d); m/z 278(M+H).

### EXAMPLE 12

A solution of sec-butyllithium in cyclohexane (22.5ml, 1.3M) was added slowly to a solution of 4-bromophenyl boronic acid-N-methyl- O:O diethanolamino ester (Tet. Let. 30, (51), 7194, 1989) (3.3g) in freshly distilled tetrahydrofuran (30ml) under an atmosphere of argon and at a temperature of -100°C.

The reaction mixture was stirred at -100°C for a further 30 minutes. A solution of quinculidin-3-one (1.45g) in freshly distilled tetrahydrofuran (6ml) was added and the reaction mixture was stirred at -100°C for 15 minutes. The reaction mixture was allowed to warm to ambient temperature and was then stirred at ambient temperature for 2 hours. A solution of ammonium chloride (1.15g) in water (10ml) was then added to the mixture. The mixture was stirred for 5 minutes and the solvent was then evaporated to give a green oil. Toluene (30ml), saturated aqueous sodium carbonate solution (15ml) and a solution of 3-bromoquinoline (2.44g) in absolute alcohol (15ml) were added to the residue. The reaction vessel was purged with argon and tetrakistriphenylphosphinepalladium [O] (200mg) was then added to the mixture. The mixture was then heated at reflux with stirring for 4 hours. The mixture was cooled to ambient temperature and the solvent evaporated. Ice followed by 2M aqueous hydrochloric acid were added to the residue and the mixture was extracted with ethyl acetate (3 x 50ml)

The aqueous phase was treated with ice, basified with aqueous sodium hydroxide solution (density 1.35g/cm³) and extracted with ethyl acetate (4 x 70ml). The ethyl acetate extracts were combined, dried (MgSO₄) and evaported. The residue was triturated with hot ethyl acetate and the mixture cooled to give 3-[4-quinol-3-ylphenyl]quinculidin-3-ol as a solid (46% yield), m.p. 220-221°C, microanalysis: found: C, 79.2; H, 6.6; N, 8.3%; C₂₂H₂₂N₂0 0.25H₂0 requires: C, 78.9; H, 6.7; N, 8.4%; NMR([CD₃]₂SO): 1.2-1.5(3H,m), 2.0(1H,br.s), 2.1-2.2(1H,m), 2.6-2.8(4H,m), 2.95(1H,d), 3.45(1H,d), 5.2(1H,s), 7.6-7.8(4H,m), 7.85(2H,d), 8.05(2H,d), 8.6(1H,d) and 9.3(1H,d); m/z 331(M+H).

### EXAMPLE 13

A solution of sec-butyllithium in cyclohexane (4.2ml, 1.3M) was added slowly to a stirred solution of 3-[4-iodophenyl]-5-methyl-1,2,4-oxadiazole (1.43g) in freshly distilled tetrahydrofuran (20ml) -100°C and under an atmosphere of argon. The reaction mixture was stirred at -100°C for 10 minutes. A solution of quinculidin-3-one (625mg) in freshly distilled tetrahydrofuran (7ml) was added.

The mixture was stirred at -100°C for 30 minutes, allowed to warm to ambient temperature and then stirred at ambient temperature for a further 2 hours. The solvent was removed by evaporation. 2M aqueous hydrochloric acid (20ml) was added and the mixture was extracted with ethyl acetate [3 x 20ml]. The aqueous phase was then basified with aqueous sodium hydroxide solution and the mixture extracted with ethyl acetate [6 x 30ml). The ethyl acetate extracts were combined, dried (MgSO₄) and evaporated. The residue was purified by preparative thin layer chromatography on silica gel plates (40 x 20 x 0.5mm; Schliecher and Schull G 1505/LS 254) using a mixture of methanol [100 parts] and ammonia (density, 0.88g/cm³) [2 parts] to give 3-[4-(5-methyl-1,2,4- oxadiazol-3-yl)phenyl]quinuclidin-3-ol (9% yield); microanalysis, found: C, 67.0; H, 6.3; N, 14.6%; C₁₆H₁₉N₃0₂ requires: C, 67.3; H, 6.7; N; 14.7%; NMR([CD₃]₂SO): 1.2-1.5(3H,m), 2.0(1H br.s), 2.1-2.2(1H,m), 2.7(3H,s), 2.6-2.8(4H,m), 2.95(1H,d), 3.45(1H,d), 5.3(1H,s), 7.7(2H,d) and 7.95(2H,d), m/z 286 (M+H).

### EXAMPLE 14

A mixture of 3-(4-bromophenyl)quinuclidin-3-ol (0.57g), 3-thienyl boronic acid (CA 52:1136g) (0.465g), tetra kis(triphenylphosphine) palladium (O) (25mg), 1,2-dimethoxy ethane (25ml) and saturated sodium bicarbonate solution (10ml) was heated at 80°C for 17 hours under an atmosphere of argon. The reaction mixture was evaporated and the residue partitioned between water (25ml) and ethyl acetate (4 x 30ml). The organic extracts were combined, washed with 2M aqueous sodium hydroxide solution (3 x 25ml), water, brine, dried (MgSO₄) and evaporated. The residue was purified by flash column chromatography on silica-gel using a 90:10 (v/v) mixture of methylene chloride/methanol as eluent to give a foam (0.5g) which was crystallised from ethyl acetate to give 3-[4-(3-thienyl)phenyl]quinuclidin-3-ol as a solid, m.p. 163-5°C; microanalysis, found: C, 70.2; H, 6.8; N, 4.8%; C₁₇H₁₉NOS 0.05 CH₂Cl₂ requires: C, 70.4; H, 6.6; N, 4.8%; NMR ([CD₃]₂SO): 1.15-2.22 (5H,m), 2.55-3.41(6H,m), 5.10(1H,br s), 7.50-7.7(6H,m) and 7.80(1H,d of d); m/z 286(M+H).

The 3-(4-bromophenyl)quinuclidin-3-ol was prepared as follows.

A suspension of magnesium turnings (12.0g) in anhydrous ether (500ml) was treated with a few crystals of iodine and dropwise with a solution of 1,4-dibromobenzene (118g) in anhydrous ether (300ml). Stirring was continued for 1 hour until the Grignard reagent had completely formed. The mixture was cooled to 5°C and a solution of 3-quinuclidone (56.2g) in tetrahydrofuran (150ml) was added portionwise. After stirring for a further 2 hours at room temperature, the reaction mixture was poured onto ice (300g) and a saturated solution of ammonium chloride (500ml), The mixture was extracted with ether (4 x 200ml). The ether extracts were combined, dried (MgS04), and evaporated. The solid residue was triturated with ethyl acetate to give 3-(4-bromopenyl)quinuclidin-3-ol as a solid, m.p. 188-191°C; NMR ([CD₃]₂SO): 1.1-1.50(3H,m), 1.87(1H,m), 2.13(1H,m), 2.55-2.83(4H,m), 2.84-2.90(1H,d), 3.25-3.40(1H, d of d), 5.18(1H,s), and 7.4-7.55(4H,m).

### EXAMPLE 15

In a similar manner to that described in Example 14 but using 3-furylboronic acid as starting material there was obtained 3-[4-(3-furyl)phenyl]quinuclidin-3-ol as a solid, m.p. 174-7°C; microanalysis, found: C, 73.4; H, 7.0; N, 5.0; H20 0.17% C₁₇H₁₉NO₂ 0.1 CH₂Cl₂ 0.05 H₂0 requires: C, 73.2, H, 6.9, N, 5.0%; H₂0 0.3% NMR ([CD₃]₂SO): 1.15-2.14(5H,m), 2.6-3.6(6H,m), 5.12(1H,br s), 6.92(1H, d of d), 7.48-7.58(4H,m), 7.7(1H, d of d) and 8.13(1H, d of d); m/z 270(M+H).

3-furylboronic acid is reported in J. Het. Chem., 12, (1975), 195; 13, (1976), 1265 and Bull. Soc. Chim. (France), (1976), 1999.

### EXAMPLE 16

Illustrative pharmaceutical dosage forms suitable for presenting the compounds of the invention for therapeutic or prophylactic use include the following tablet and capsule formulations, which may be obtained by conventional procedures well known in the art of pharmacy and are suitable for therapeutic or prophylactic use in humans:-

| (a) Tablet I | |
|---|---|
| | mg/tablet |
| Compound Z^{∗} | 1.0 |
| Lactose Ph. Eur. | 93.25 |
| Croscarmellose sodium | 4.0 |
| Maize starch paste (5% w/v aqueous paste) | 0.75 |
| Magnesium stearate | 1.0 |

| (b) Tablet II mg/tablet | |
|---|---|
| Compound Z^{∗} | 50 |
| Lactose Ph. Eur | 223.75 |
| Croscarmellose sodium | 6.0 |
| Maize starch.0 | |
| Polyvinylpyrrolidone (5% w/v aqueous paste) | 2.25 |
| Magnesium stearate | 3.0 |

| (c) Tablet III | |
|---|---|
| | mg/tablet |
| Compound Z^{∗} | 100 |
| Lactose Ph. Eur. | 182.75 |
| Croscarmellose sodium | 12.0 |
| Maize starch paste (5% w/v aqueous paste) | 2.25 |
| Magnesium stearate | 3.0 |

| (d) Capsule | |
|---|---|
| | mg/capsule |
| Compound Z∗ | 10 |
| Lactose Ph.Eur. | 488.5 |
| Magnesium stearate | 1.5 |

| | |
|---|---|
| Note ∗ The active ingredient Compound Z is a compound of formula I, or a salt thereof, for example a compound of formula I described in any of the preceding Examples. | |

The tablet compositions (a) - (c) may be enteric coated by conventional means, for example, with cellulose acetate phthalate

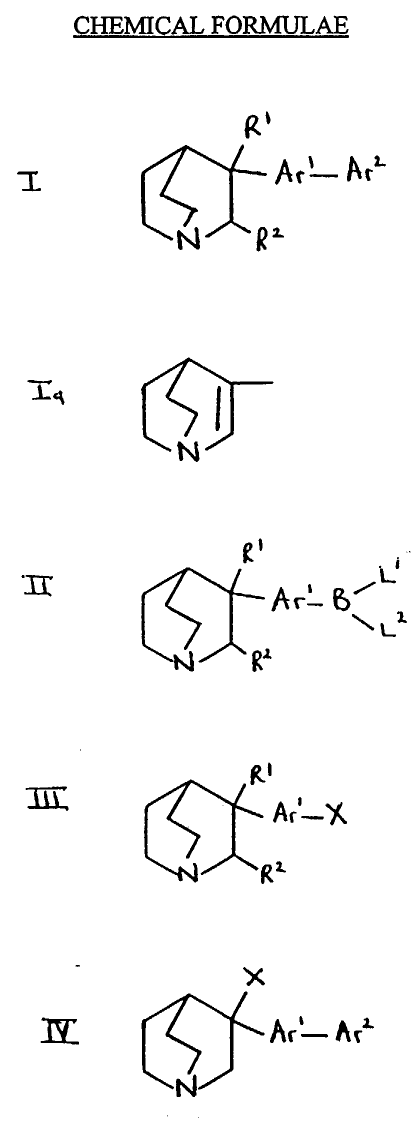

## Claims

1. A compound of formula I: 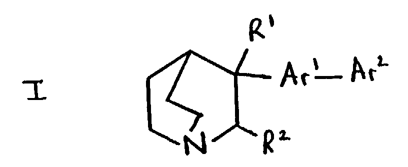 or a pharmaceutically acceptable salt thereof, wherein:
R¹ is hydrogen or hydroxy;
R² is hydrogen; or
R¹ and R² are joined together so that CR¹-CR² is a double bond;
Ar¹ is a phenylene moiety and Ar² is a heterocyclic moiety selected from an aromatic 5-membered or 6-membered heterocyclic ring containing one, two or three heteroatoms selected from nitrogen, oxygen and sulphur; an aromatic 5-membered or 6-membered heterocyclic ring containing one, two or three heteroatoms selected from nitrogen, oxygen and sulphur which is fused to a benzene ring; and a bicyclic heterocycle which consists of a non-aromatic 5-membered or 6-membered heterocyclic ring containing (in addition to carbon atoms) one, two or three heteroatoms selected from nitrogen, oxygen and sulphur fused to a benzene ring;
and wherein one or both of Ar¹ and Ar² may optionally bear one or more substituents independently selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino N-(1-6C)alkylcarbamoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno (1-6C)alkyl, (1-6C)alkanoylamino, ureido, N'-(1-6C)alkylureido, (1-6C)alkanoyl and oxime derivatives thereof and O-(1-6C)alkyl ethers of said oxime derivatives; provided that Ar² is not a 6-membered heteroaryl moiety containing one or two nitrogen atoms.

2. A compound as claimed in claim 1 wherein one or both of Ar¹ and Ar² may optionally be unsubstituted or may bear one or more substituents independently selected from halogeno, hydroxy, amino, nitro, (1-6C)alkyl, (2-6C)alkenyl, (1-6C)alkoxy, (1-6C)alkanoyl and oxime derivatives thereof and O-(1-6C)alkyl ethers of said oximes, halogeno(1-6C)alkyl and (1-6C)alkanoylamino.

3. A compound as claimed in claim 1 or 2 wherein Ar¹ is a 1,4-phenylene moiety and Ar² is a heterocyclic moiety selected from a pyrrolyl, quinolyl, oxadiazolyl, oxazolyl, thienyl, thiazolyl, furyl, pyridyl, benzthiazolyl, benzoxazolyl, benzimidazolyl and benzdioxolyl.

4. A compound as claimed in claim 3 wherein Ar² is a heterocyclic moiety selected from pyrrolyl, quinolyl, oxadiazolyl, oxazolyl, thiazolyl, benzthiazolyl and a benzdioxolyl moiety.

5. compound as claimed in any one of the preceeding claims wherein R¹ is hydroxy and R² is hydrogen.

6. A compound of formula I, or a pharmaceutically acceptable salt thereof, as claimed in claim 1 wherein:
R¹ is hydroxy; R² is hydrogen; Ar¹ is a phenylene moiety and Ar² is a heterocyclic moiety selected from an aromatic 5-membered or 6-membered heterocyclic ring containing one, two or three heteroatoms selected from nitrogen, oxygen and sulphur; an aromatic 5-membered or 6-membered heterocyclic ring containing one, two or three heteroatoms selected from nitrogen, oxygen and sulphur which is fused to a benzene ring; and a bicyclic heterocycle which consists of a non-aromatic 5-membered or 6-membered heterocyclic ring containing (in addition to carbon atoms) one, two or three heteroatoms selected from nitrogen, oxygen and sulphur fused to a benzene ring;
and wherein one or both of Ar¹ and Ar² may optionally bear one or more substituents independently selected from halogeno, hydroxy, amino, nitro, cyano, carboxy, carbamoyl, (1-6C)alkyl, (2-6C)alkenyl, (2-6C)alkynyl, (1-6C)alkoxy, (1-6C)alkylamino, di-[(1-6C)alkyl]amino N-(1-6C)alkylcarbamoyl, di-N,N-[(1-6C)alkyl]carbamoyl, (1-6C)alkoxycarbonyl, (1-6C)alkylthio, (1-6C)alkylsulphinyl, (1-6C)alkylsulphonyl, halogeno (1-6C)alkyl, (1-6C)alkanoylamino, ureido, N'-(1-6C)alkylureido, (1-6C)alkanoyl and oxime derivatives thereof and O-(1-6C)alkyl ethers of said oxime derivatives; provided that Ar² is not a 6-membered heteroaryl moiety containing one or two nitrogen atoms.

7. A compound as claimed in claim 6 wherein Ar¹ is a 1,4-phenylene moiety and Ar² is selected from an oxadiazole and a quinolyl moiety.

8. A compound as claimed in claim 1 which is selected from
3-[4-(3-methyl-1,2,4-oxadiazol-5-yl)phenyl)-quinuclidin-3-ol;
and pharmaceutically acceptable salts thereof

9. A process for preparing a compound of formula I, or a pharmaceuticaly acceptable salt thereof, as claimed in claim 1 which process is selected from:
(a) for those compounds of formula I in which R¹ is hydroxy and R² is hydrogen, reacting a compound of formula Ar²-Ar¹-M in which M is a metal atom or a derivative thereof, with quinuclidin-3-one;
(b) reacting a compound of formula II: 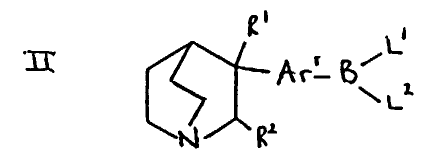 wherein L¹ and L2 are suitable ligands with a compound of formula Ar²-X, in which X represents a leaving group, in the presence of a catalyst;
(c) reacting a compound of formula III: 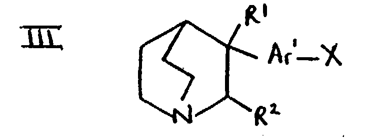 in which X is a suitable leaving group with a compound of formula hal-Ar¹-B(L¹)L² in which L¹ and L² are suitable ligands in the presence of a catalyst;
(d) for those compounds of formula I in which R¹ and R² are both hydrogen, reducing a compound of formula I in which R¹ and R² are joined together so that -CR¹-CR² is a double bond;
(e) for those compounds of formula I in which R¹ and R² are joined together so that CR¹-CR² is a double bond, dehydrating a compound of formula I in which R¹ is hydroxy;
(f) for compounds of formula I in which R¹ and R² are joined together so that CR¹-CR² is a double bond, treating a compound of formula IV: 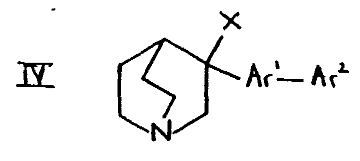 in which X is a leaving group with a base; and
(g) for those compounds of formula I in which R¹ and R² are both hydrogen, by dehydroxylation of a compound of formula I in which R¹ is hydroxy;
and whereafter, when a pharmaceutically-acceptable salt of a compound of the formula I is required, reacting a compound of formula I with an acid which affords a physiologically acceptable anion, or with a base which affords a physiologically acceptable cation.

10. A pharmaceutical composition comprising a compound of formula I, or a pharmaceutically acceptable salt thereof thereof, as claimed in any one of claims 1 to 8 together with a pharmaceutically acceptable diluent or carrier.

11. The use of a compound of formula I, or a pharmaceutically acceptable salt thereof, as claimed in any one of claims 1 to 8 for the manufacture of a medicament for inhibiting cholesterol biosynthesis.

12. The use as claimed in claim 11 for the manufacture of a medicament for treating hypercholeserolemia and/or atherosclerosis.

## Patentansprüche

1. Verbindung der Formel I: 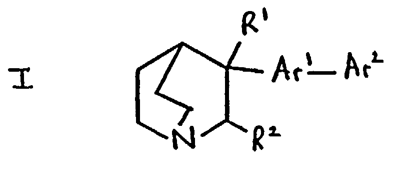 oder ein pharmazeutisch akzeptables Salz davon, worin:
R¹ Wasserstoff oder Hydroxy ist;
R² Wasserstoff ist; oder
R¹ und R² miteinander verbunden sind, so daß CR¹-CR² eine Doppelbindung ist;
Ar¹ eine Phenylen-Einheit ist und Ar² eine heterocyclische Einheit ist, ausgewählt aus einem aromatischen 5-gliedrigen oder 6-gliedrigen heterocyclischen Ring, der 1, 2 oder 3 Heteroatome enthält, ausgewählt aus Stickstoff, Sauerstoff und Schwefel; einem aromatischen 5-gliedrigen oder 6-gliedrigen heterocyclischen Ring, der 1, 2 oder 3 Heteroatome enthält, ausgewählt aus Stickstoff, Sauerstoff und Schwefel, der an einen Benzol-Ring kondensiert ist; und einem bicyclischen Heterocyclus, der aus einem nicht-aromatischen 5-gliedrigen oder 6-gliedrigen heterocyclischen Ring besteht, der (zusätzlich zu Kohlenstoffatomen) 1, 2 oder 3 Heteroatome enthält, ausgewählt aus Stickstoff, Sauerstoff und Schwefel, und an einen Benzol-Ring kondensiert ist;
und worin eines oder beide aus Ar¹ und Ar² gegebenenfalls einen oder mehrere Substituenten tragen können, die unabhängig ausgewählt sind aus Halogen, Hydroxy, Nitro, Cyano, Carboxy, Carbamoyl, (C₁₋₆)-Alkyl, (C₂₋₆)-Alkenyl, (C₂₋₆)-Alkinyl, (C₁₋₆)-Alkoxy, (C₁₋₆)-Alkylamino, Di-[(C₁₋₆)-alkyl]amino-N-(C₁₋₆)-alkylcarbamoyl, Di-N,N-[(C₁₋₆)-alkyl]carbamoyl, (C₁₋₆)-Alkoxycarbonyl, (C₁₋₆) -Alkylthio, (C₁₋₆)-Alkylsulfinyl, (C₁₋₆) -Alkylsulfonyl, Halogen-(C₁₋₆)-alkyl, (C₁₋₆)-Alkanoylamino, Ureido, N'-(C₁₋₆)-Alkylureido, (C₁₋₆)-Alkanoyl und Oxim-Derivaten davon und O-(C₁₋₆)-Alkylethern der Oxim-Derivate;
mit der Maßgabe, daß Ar² keine 6-gliedrige Heteroaryl-Einheit ist, die 1 oder 2 Stickstoffatome enthält.

2. Verbindung gemäß Anspruch 1, worin eines oder beide aus Ar¹ und Ar² gegebenenfalls unsubstituiert sein können oder einen oder mehrere Substituenten tragen können, die unabhängig ausgewählt sind aus Halogen, Hydroxy, Amino, Nitro, (C₁₋₆)-Alkyl, (C₂₋₆) -Alkenyl, (C₁₋₆)-Alkoxy, (C₁₋₆)-Alkanoyl und Oxim-Derivaten davon und O-(C₁₋₆)-Alkylethern der Oxime, Halogen-(C₁₋₆)-alkyl und (C₁₋₆)-Alkanoylamino.

3. Verbindung gemäß Anspruch 1 oder 2, worin Ar¹ eine 1,4- Phenylen-Einheit ist und Ar² eine heterocyclische Einheit ist, ausgewählt aus Pyrrolyl, Chinolyl, Oxadiazolyl, Oxazolyl, Thienyl, Thiazolyl, Furyl, Pyridyl, Benzthiazolyl, Benzoxazolyl, Benzimidazolyl und Benzdioxolyl.

4. Verbindung gemäß Anspruch 3, worin Ar² eine heterocyclische Einheit ist, ausgewählt aus Pyrrolyl, Chinolyl, Oxadiazolyl, Oxazolyl, Thiazolyl, Benzthiazolyl und einer Benzdioxolyl-Einheit.

5. Verbindung gemäß einem der vorhergehenden Ansprüche, worin R¹ Hydroxy ist und R² Wasserstoff ist.

6. Verbindung der Formel I oder ein pharmazeutisch akzeptables Salz davon gemäß Anspruch 1, worin:
R¹ Hydroxy ist; R² Wasserstoff ist; Ar¹ eine Phenylen-Einheit ist und Ar² eine heterocyclische Einheit ist, ausgewählt aus einem aromatischen 5-gliedrigen oder 6-gliedrigen-heterocyclischen Ring, der 1, 2 oder 3 Heteroatome enthält, ausgewählt aus Stickstoff, Sauerstoff und Schwefel; einem aromatischen 5-gliedrigen oder 6-gliedrigen heterocyclischen Ring, der 1, 2 oder 3 Heteroatome enthält, ausgewählt aus Stickstoff, Sauerstoff und Schwefel, der an einen Benzol-Ring kondensiert ist; und einem bicyclischen Heterocyclus, der aus einem nicht-aromatischen 5-gliedrigen oder 6-gliedrigen heterocyclischen Ring besteht, der (zusätzlich zu Kohlenstoffatomen) 1, 2 oder 3 Heteroatome enthält, ausgewählt aus Stickstoff, Sauerstoff und Schwefel, und an einen Benzol-Ring kondensiert ist;
und worin eines oder beide aus Ar¹ und Ar² gegebenenfalls einen oder mehrere Substituenten tragen können, unabhängig ausgewählt aus Halogen, Hydroxy, Amino, Nitro, Cyano, Carboxy, Carbamoyl, (C₁₋₆)-Alkyl, (C₂₋₆) -Alkenyl, (C₂₋₆)-Alkinyl, (C₁₋₆)-Alkoxy, (C₁₋₆)-Alkylamino, Di-[(C₁₋₆)-alkyl]amino-N-(C₁-₆)-alkylcarbamoyl, Di-N,N- [(C₁₋₆)-alkyl]carbamoyl, (C₁₋₆)-Alkoxycarbonyl, (C₁₋₆)-Alkylthio, (C₁₋₆)-Alkylsulfinyl, (C₁₋₆) -Alkylsulfonyl, Halogen-(C₁₋₆)-alkyl, (C₁₋₆)-Alkanoylamino, Ureido, N'-(C₁-₆)-Alkylureido, (C₁₋₆)-Alkanoyl und Oxim-Derivaten davon und O-(C₁₋₆)-Alkylethern der Oxim-Derivate;
mit der Maßgabe, daß Ar² keine 6-gliedrige Heteroaryl-Einheit ist, die 1 oder 2 Stickstoffatome enthält.

7. Verbindung gemäß Anspruch 7, worin Ar¹ eine 1,4- Phenylen-Einheit ist und Ar² aus einer Oxadiazol- und einer Chinolyl-Einheit ausgewählt ist.

8. Verbindung gemäß Anspruch 1, die ausgewählt ist aus 3- [4-(3-Methyl-1,2,4-oxadiazol-5-yl)phenyl)chinuclidin-3-ol; und pharmazeutisch akzeptable Salze davon.

9. Verfahren zur Herstellung einer Verbindung der Formel I oder eines pharmazeutisch akzeptablen Salzes davon gemäß Anspruch 1, wobei das Verfahren ausgewählt ist aus:
(a) für diejenigen Verbindungen der Formel I, worin R¹ Hydroxy ist und R² Wasserstoff ist, Umsetzen einer Verbindung der Formel Ar²-Ar¹-M, worin M ein Metallatom ist, oder eines Derivats davon mit Chinuclidin-3-on;
(b) Umsetzen einer Verbindung der Formel II: 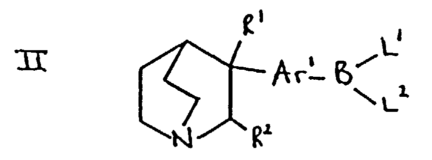 worin L¹ und L² geeignete Liganden sind, mit einer Verbindung der Formel Ar²-X, worin X eine Abgangsgruppe darstellt, in Gegenwart eines Katalysators;
(c) Umsetzen einer Verbindung der Formel III: 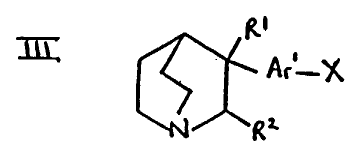 worin X eine geeignete Abgangsgruppe ist, mit einer Verbindung der Formel Hal-Ar¹-B(L¹)L², worin L¹ und L² geeignete Liganden sind, in Gegenwart eines Katalysators;
(d) für diejenigen Verbindungen der Formel I, worin R¹ und R² beide Wasserstoff sind, Reduzieren einer Verbindung der Formel I, worin R¹ und R² miteinander verbunden sind, so daß -CR¹-CR² eine Doppelbindung ist;
(e) für diejenigen Verbindungen der Formel I, worin R¹ und R² miteinander verbunden sind, so daß CR¹-CR² eine Doppelbindung ist, Dehydratisieren einer Verbindung der Formel I, worin R¹ Hydroxy ist;
(f) für Verbindungen der Formel I, worin R¹ und R² miteinander verbunden sind, so daß CR¹-CR² eine Doppelbindung ist, Behandeln einer Verbindung der Formel IV: 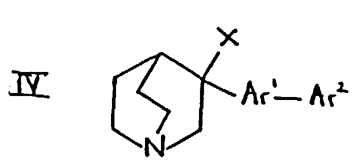 worin X eine Abgangsgruppe ist, mit einer Base; und
(g) für diejenigen Verbindungen der Formel I, worin R¹ und R² beide Wasserstoff sind, durch Dehydroxylierung einer Verbindung der Formel I, worin R¹ Hydroxy ist;
und danach, wenn ein pharmazeutisch akzeptables Salz einer Verbindung der Formel I erforderlich ist, Umsetzen einer Verbindung der Formel I mit einer Säure, die ein physiologisch akzeptables Anion liefert, oder mit einer Base, die ein physiologisch akzeptables Kation liefert.

10. Pharmazeutische Zusammensetzung, umfassend eine Verbindung der Formel I oder ein pharmazeutisch akzeptables Salz davon gemäß einem der Ansprüche 1 bis 9 zusammen mit einem pharmazeutisch akzeptablen Verdünnungsstoff oder Träger.

11. Verwendung einer Verbindung der Formel I oder eines pharmazeutisch akzeptablen Salzes davon gemäß einem der Ansprüche 1 bis 9 zur Herstellung eines Medikaments zur Inhibierung der Cholesterol-Biosynthese.

12. Verwendung gemäß Anspruch 12 zur Herstellung eines Medikaments zur Behandlung von Hypercholesterinämie und/oder Atherosklerose.

## Revendications

1. Composé de formule (I): 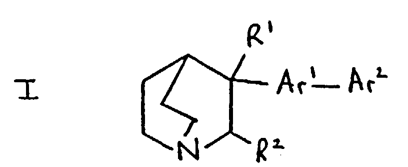 ou sel pharmaceutiquement acceptable de celui-ci, dans laquelle
- R¹ représente un hydrogène ou un groupe hydroxy ;
- R² représente un hydrogène ; ou
- R¹ et R² sont unis l'un à l'autre de sorte que CR¹⁻CR² est une double liaison ;
- Ar¹ représente une entité phénylène et Ar² représente une entité hétérocyclique choisie parmi un hétérocycle aromatique à 5 atomes ou à 6 atomes contenant un, deux ou trois hétéroatomes choisis parmi l'azote, l'oxygène et le soufre ; un hétérocycle aromatique à 5 atomes ou à 6 atomes contenant un, deux ou trois hétéroatomes choisis parmi l'azote, l'oxygène et le soufre et qui est fusionné à un cycle benzénique ; et un hétérocycle bicyclique qui consiste en un hétérocycle non aromatique à 5 atomes ou à 6 atomes contenant (en plus des atomes de carbone), un, deux ou trois hétéroatomes choisis parmi l'azote, l'oxygène et le soufre, fusionné à un cycle benzénique ;
et dans laquelle l'un des groupes Ar¹ ou Ar² ou les deux, peuvent éventuellement porter un ou plusieurs substituants choisis indépendamment parmi les groupes halogéno, hydroxy, amino, nitro, cyano, carboxy, carbamoyle, alkyle(1-6C), alkényle(2-6C), alkynyle(2-6C), alcoxy(1-6C), alkyl(1-6C)amine, di-[alkyl(1-6C)]amine N-alkyl(1-6C)carbamoyle, di-N,N-[alkyl(1-6C)]carbamoyle, alcoxy(1-6C)carbonyle, alkyl(1-6C)thio, alkyl(1-6C)sulfinyle, alkyl(1-6C)sulfonyle, halogénoalkyle (1-6C), alca-noyl(1-6C)amino, uréido, N'-alkyl(l-6C)alkyluréido, alcanoyle (1-6C) et les dérivés oxime de ceux-ci et les éthers O-alkyle (1-6C) desdits dérivés oxime ; à la condition que Ar² ne soit pas une entité hétéroaryle à 6 atomes contenant un ou deux atomes d'azote.

2. Composé selon la revendication 1, dans lequel l'un des groupes Ar¹ ou Ar² ou les deux, peuvent éventuellement être non substitués ou peuvent porter un ou plusieurs substituants choisis indépendamment parmi les groupes halogéno, hydroxy, amino, nitro, alkyle (1-6C), alkényle (2-6C), alcoxy (1-6C), alcanoyle (1-6C) et les dérivés oxime de ceux-ci et les éthers O-alkyle (1-6C) desdits dérivés oxime, halogénoalkyle (1-6C) et alcanoyl(1-6C)amino.

3. Composé selon la revendication 1 ou la revendication 2 dans lequel Ar¹ est une entité 1,4-phénylène et Ar² est une entité hétérocyclique choisie parmi les entités pyrrolyle, quinolyle, oxadiazolyle, oxazolyle, thiényle, thiazolyle, furyle, pyridyle, benzthiazolyle, benzoxa-zolyle, benzimidazolyle et benzdioxolyle.

4. Composé selon la revendication 3, dans lequel Ar² est une entité hétérocyclique choisie parmi les entités pyrrolyle, quinolyle, oxadiazolyle, oxazolyle, thiazolyle, benzthiazolyle et benzdioxolyle.

5. Composé selon l'une quelconque des revendications précédentes, dans lequel R¹ est un hydroxy et R² est un hydrogène.

6. Composé de formule (I) ou sel pharmaceutiquement acceptable de celui-ci, dans laquelle :
R¹ représente un hydroxy ; R² représente un hydrogène ; Ar¹ représente une entité phénylène et Ar² représente une entité hétérocyclique choisie parmi un hétérocycle aromatique à 5 atomes ou à 6 atomes contenant un, deux ou trois hétéroatomes choisis parmi l'azote, l'oxygène et le soufre ; un hétérocycle aromatique à 5 atomes ou à 6 atomes contenant un, deux ou trois hétéroatomes choisis parmi l'azote, l'oxygène et le soufre et qui est fusionné à un cycle benzénique ; et un hétérocycle bicyclique qui consiste en un hétérocycle non aromatique à 5 atomes ou à 6 atomes contenant (en plus des atomes de carbone), un, deux ou trois hétéroatomes choisis parmi l'azote, l'oxygène et le soufre, fusionné à un cycle benzénique ;
et dans laquelle l'un des groupes Ar¹ ou Ar² ou les deux, peuvent éventuellement porter un ou plusieurs substituants choisis indépendamment parmi les groupes halogéno, hydroxy, amino, nitro, cyano, carboxy, carbamoyle, alkyle(1-6C), alkényle(2-6C), alkynyle(2-6C), alcoxy(1-6C), alkyl( 1-6C)amino, di-[alkyl( -6C)]amino N-alkyl( -6C)carbamoyle, di-N,N-[alkyl(1-6C)]carbamoyle, alcoxy(1-6C)carbonyle, alkyl(1-6C)thio, alkyl(1-6C)sulfinyle, alkyl(1-6C)sulfonyle, halogénoalkyle (1-6C), alca-noyl(1-6C)amino, uréido, N'-alkyl(1-6C)alkyluréido, alcanoyle (1-6C) et les dérivés oxime de ceux-ci et les éthers O-alkyle (1-6C) desdits dérivés oxime ; à la condition que Ar² ne soit pas une entité hétéroaryle à 6 atomes contenant un ou deux atomes d'azote.

7. Composé selon la revendication 7, dans lequel Ar¹ est une entité 1,4-phénylène et Ar² est choisi parmi les entités oxadiazole et quinolyle.

8. Composé selon la revendication 1, qui est choisi parmi le 3-[4-(3-méthyl-1,2,4-oxadiazol-5-yl)phényl]quinuclidin-3-ol et les sels pharmaceutiquement acceptables de celui-ci.

9. Procédé pour la préparation d'un composé de formule (I) ou d'un sel pharmaceutiquement acceptable de celui-ci selon la revendication 1, lequel procédé est choisi entre :
(a) pour les composés de formule (I) dans laquelle R¹ est un hydroxy et R² est un hydrogène, la réaction d'un composé de formule Ar²-Ar¹-M dans laquelle M est un atome de métal ou un dérivé de celui-ci, avec la quinuclidin-3-one ;
(b) la réaction d'un composé de formule (II) 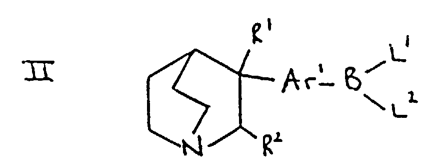 dans laquelle L¹ et L² sont des ligands appropriés, avec un composé de formule Ar²⁻X dans laquelle X représente un groupe partant, en présence d'un catalyseur ;
(c) la réaction d'in composé de formule (III) : 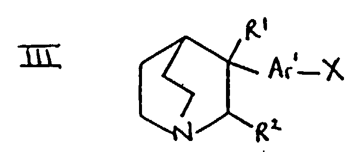 dans laquelle X est groupe partant approprié, avec un composé de formule hal-Ar¹-B(L¹)L² dans laquelle L¹ et L² sont des ligands appropriés, en présence d'un catalyseur ;
(d) pour les composés de formule (I) dans laquelle R¹ et R² représentent tous deux l'hydrogène, la réduction d'un composé de formule (I) dans laquelle R¹ et R² sont unis l'un à l'autre de sorte que -CR¹ -CR² est une double liaison ;
(e) pour les composés de formule (I) dans laquelle R¹ et R² sont unis l'un à l'autre de sorte que -CR¹ -CR² représente une double liaison, la déshydratation d'un composé de formule (I) dans laquelle R¹ est un hydroxy ;
(f) pour les composés de formule (I) dans laquelle R¹ et R² sont unis l'un à l'autre de sorte que -CR¹-CR² représente une double liaison, le traitement d'un composé de formule (IV) : 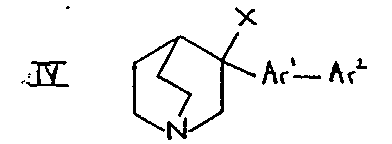 dans laquelle X est un groupe partant, avec une base ; et
(g) pour les composés de formule (I) dans laquelle R¹ et R² représentent tous deux l'hydrogène, la déshydroxylation d'un composé de formule (I) dans laquelle R¹ est un hydroxy ;
et après cela, lorsqu'un sel pharmaceutiquement acceptable d'un composé de formule (I) est nécessaire, la réaction d'un composé de formule (I) avec un acide qui apporte un anion physiologiquement acceptable ou avec une base qui apporte un cation physiologiquement acceptable.

10. Composition pharmaceutique comprenant un composé de formule (I) ou un sel pharmaceutiquement acceptable de celui-ci selon l'une quelconque des revendications 1 à 9, en association avec un diluant ou véhicule pharmaceutiquement acceptable.

11. Utilisation d'un composé de formule (I) ou d'un sel pharmaceutiquement acceptable de celui-ci selon l'une quelconque des revendications 1 à 9, pour la fabrication d'un médicament destiné à inhiber la biosynthèse du cholestérol.

12. Utilisation selon la revendication 12, pour la fabrication d'un médicament destiné au traitement de l'hypercholestérolémie et/ou de l'artériosclérose.
